(19) Europäisches Patentamt
European Patent Office
Office européen des brevets

(11) EP 1 500 645 B1

(12) **EUROPEAN PATENT SPECIFICATION**

(45) Date of publication and mention of the grant of the patent:
**15.07.2009 Bulletin 2009/29**

(21) Application number: **03728006.2**

(22) Date of filing: **28.04.2003**

(51) Int Cl.:
*C07C 403/24* (2006.01) *C12P 23/00* (2006.01)
*A23L 1/30* (2006.01) *A61Q 1/04* (2006.01)

(86) International application number:
**PCT/JP2003/005443**

(87) International publication number:
**WO 2003/093229 (13.11.2003 Gazette 2003/46)**

(54) **ASTAXANTHIN MEDIUM-CHAIN FATTY ACID ESTER, PROCESS FOR PRODUCING THE SAME AND COMPOSITION CONTAINING THE ESTER**

ASTAXANTHIN-FETTSÄUREESTER MIT MITTLERER KETTENLÄNGE, VERFAHREN ZU DESSEN HERSTELLUNG UND ZUSAMMENSETZUNG, DIE DEN ESTER ENTHÄLT

ESTERS D'ACIDES GRAS A CHAINE MOYENNE ASTAXANTHINE, LEUR PROCEDE DE PRODUCTION ET COMPOSITION RENFERMANT CE ESTER

(84) Designated Contracting States:
**AT BE BG CH CY CZ DE DK EE ES FI FR GB GR HU IE IT LI LU MC NL PT RO SE SI SK TR**

(30) Priority: **30.04.2002 JP 2002128989**

(43) Date of publication of application:
**26.01.2005 Bulletin 2005/04**

(73) Proprietor: **Suntory Holdings Limited**
**Kita-ku Osaka-shi**
**Osaka 530-8203 (JP)**

(72) Inventors:
- **SUMIDA, Motoo**
  **Uji-shi, Kyoto 611-0043 (JP)**
- **NAKAO, Masahiro**
  **Nagaokakyo-shi, Kyoto 617-0827 (JP)**
- **TOMIMORI, Namino**
  **Ibaraki-shi, Osaka 567-0833 (JP)**
- **NAMIKAWA, Koshi**
  **Hirakata-shi, Osaka 573-0092 (JP)**
- **FUKAMI, Harukazu**
  **Kyoto-shi, Kyoto 601-8373 (JP)**

(74) Representative: **Vossius & Partner**
**Siebertstrasse 4**
**81675 München (DE)**

(56) References cited:
**WO-A-00/62625** **JP-A- 1 202 261**
**JP-A- 7 300 421** **JP-A- 11 290 094**

- **VINCENT M. ET AL.: 'Variation de la composition en acides gras des monoesters de zeaxanthine et d' astaxanthine dans l'ovare et l'hepatopancresa de panaeus schmitti au cours de l'ovogenese' ARCHIVES INTERNATIONALES DE PHYSIOLOGIE ET DE BIOCHIMIE vol. 97, no. 1, 1989, pages 71 - 78, XP002971825**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 1 500 645 B1

## Description

### TECHNICAL FIELD

The present invention relates to a method of producing an astaxanthin medium-chain fatty acid monoester or astaxanthin medium-chain fatty acid diester, and a composition comprising the same. More specifically, the present invention relates to a method of producing an astaxanthin medium-chain fatty acid monoester or astaxanthin medium-chain fatty acid diester using esterification or transesterification of lipase reaction. In this lipase reaction, substrates are a free astaxanthin or an astaxanthin fatty acid ester and/or mixture of a free and fatty acid ester form astaxanthins, and the donor of fatty acid residue are a free medium-chain fatty acid and/or triglyceride form of medium-chain fatty acid. And more, the present invention relates to a method of producing an astaxanthin medium-chain fatty acid monoester or astaxanthin medium-chain fatty acid diester by extraction from Crustacea; and a composition comprising these.

### BACKGROUND ART

Generally, a free astaxanthin is a natural pigment represented by the following formula (1):

[Formula (1)]

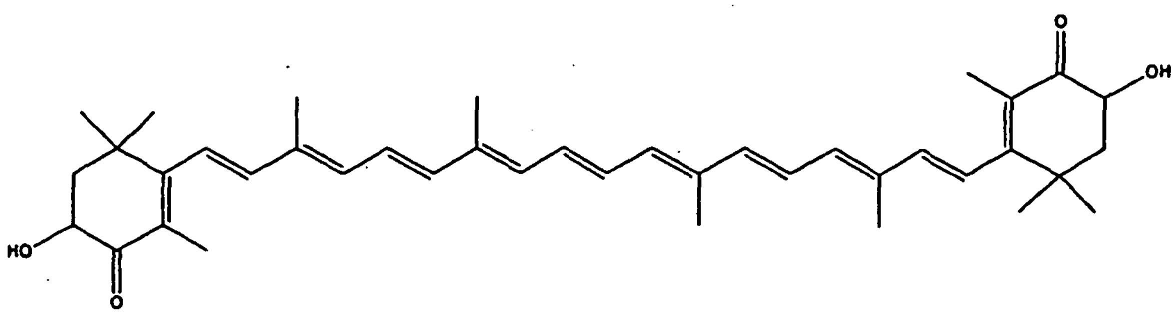

It is known that the above described free astaxanthin and an ester form of astaxanthin in which a hydroxyl group is esterified with a fatty acid are present in the nature. The fatty acid ester is usually a long chain fatty acid having 16 or more carbon atoms. These astaxanthins are classified into a monoester form and a diester form, depending on the number of fatty acids, which bind thereto.

It is known that astaxanthin is one of carotenoids and that it has remarkable antioxidant action and activity as provitamin A. For its color and physiological function (antioxidant activity), astaxanthin is used as a natural pigment, a cosmetics and a healthy food and/or supplements. For such use, astaxanthin extracted from Euphausiacea, shrimp, crab, Phaffia yeast or Chlorophyceae, Haematococcus, is used. Astaxanthin is a reddish pigment, which is found widely among Crustacea such as shrimps or crabs, the muscles or eggs of redfish or trout, the body surface of sea bream, carp or goldfish, and others. Astaxanthin has already been chemically synthesized, and the synthesized astaxanthin has been used as a feed additive for the purpose of coloring cultured fish. In the nature, astaxanthin is present as a free astaxanthin or astaxanthin fatty acid ester, or mixture of a free and ester form. Moreover, a fatty acid ester is generally present in a mixture of long chain fatty acid esters such as palmitic acid, stearic acid, oleic acid, linoleic acid, linolenic acid, EPA and DHA. Moreover, it is known that Phaffia yeast contains only a free form astaxanthin, and further, it is known that such natural astaxanthins are also present in a mixture of what is called astaxanthin long chain fatty acid esters, or as a mixture of a long chain fatty acid ester and a free astaxanthin. It is also known that astaxanthin esterified with fatty acids has much intestinal absorption rate than the free astaxanthin (Shokuhin to Kaihatsu [Food processing and ingradients], Vol. 27, No. 3, 38-40 (1992); Kagaku to Seibutsu, Vol. 28, No. 4, 219-227 (1990)). Several methods of obtaining the ester form astaxanthin have been published. That is, a method of esterifying an astaxanthin and a long chain fatty acid using lipase as a catalyst (Japanese Patent Laid-Open (JPA) No. 11-290094) and a chemically synthesizing method for palmitic acid ester of astaxanthin (Japanese Patent Laid-Open (JPA) No. 1-202261) have been published. On the other hand, it is known that medium-chain fatty acid esters such as capric acid or lauric acid are present in certain kinds of Chlorophyceae and plants, although their amount is very small. In particular, the presence of the octanoic acid ester of asthaxanthin has only been suggested, but it has not been specified as a compound (Comp. Biochem. Physiol., B: Comp. Biochem. (1987), 86B(3), 587-591). Moreover, these astaxanthin medium-chain fatty acid

esters have not been synthesized chemically or enzymatically, and accordingly, their physical properties and functions have not been examined.

DISCLOSURE OF THE INVENTION

The present invention provides, as a novel substance, an astaxanthin medium-chain fatty acid ester such as astaxanthin octanoic acid monoester or astaxanthin octanoic acid diester, which is expected to be applied in the fields of food, cosmetics and pharmaceuticals, and which has better intestinal absorption rate than an astaxanthin long chain fatty acid ester, having a high accumulation rate in the liver tissues.

The present invention further provides a method of synthesizing the astaxanthin medium-chain fatty acid ester using lipase reaction or a method of producing the medium-chain fatty acid ester by extracting it from Crustacea, preferably Euphausiacea, compositions comprising these, and food or cosmetics comprising these compositions.

BRIEF DESCRIPTION OF THE DRAWINGS

Figure 1 is a graph showing the results of measurement, which was carried out by using a commercially available astaxanthin extracted from Chlorophyceae, Haematococcus (Itano, product name: Astax9000H) and astaxanthins that are mono- and di-esterified with caprylic acid (Asta-C8-monoester and Asta-C8-diester), diluting these compounds with olive oil so as to obtain a ratio of 100 mg/kg in the conversion of a free astaxanthin, orally administering the diluted compounds to rats, and measuring the content of astaxanthin in the blood (blood plasma) of each rat by HPLC, 3, 5, 7 and 10 hours after administration

Figure 2 is a graph showing the results of the same measurement in the same experiment as in the above Figure 1, with only the exception that the content of astaxanthin in the liver was measured by HPLC;

Figure 3 shows the GC-MS results of authentic octanoic acid methyl ester; and

Figure 4 shows the GC-MS results of purified Euphausiacea samples.

BEST MODE FOR CARRYING OUT THE INVENTION

The present inventors have focused on the fact that the poor intestinal absorption rate that is considered to be a disadvantage of astaxanthin is improved in its esters. This is to say, astaxanthin long chain fatty acid esters have better intestinal absorption rate than free astaxanthin. Moreover, as a result of intensive studies directed towards finding out means for remarkably improving intestinal absorption rate, they have found that the object of the present invention can be achieved by an astaxanthin medium-chain fatty acid ester having excellent intestinal absorption rate, thereby completing the present invention.

That is to say, when an astaxanthin medium-chain fatty acid ester is orally administered, it shows better intestinal absorption rate than astaxanthins derived from Haematococcus existing in the nature (long chain fatty acid ester mixture). Moreover, when the compound is digested, astaxanthin medium-chain fatty acid esters are cleaved into free astaxanthins and free medium-chain fatty acids, and so these are digested as free forms at intestine. It is well known that even if the medium-chain fatty acid that is released at this time is incepted in a large amount, it is decomposed in the body and converted into energy, not being accumulated as body fat. Accordingly, this well fits the recently increased health orientation.

Moreover, the astaxanthin medium-chain fatty acid ester obtained by the present invention is not only provided as a novel substance, but also a composition containing the present astaxanthin medium-chain fatty acid ester can widely be applied, as an alternative of commercially available natural astaxanthins, to food, food additives, cosmetics and others, because the present astaxanthin medium-chain fatty acid ester has better intestinal absorption rate and accumulates in liver tissue penetration much better than commercially available natural astaxanthins.

Astaxanthin medium-chain fatty acid ester

The term, astaxanthin medium-chain fatty acid ester, is used in the present specification to mean a compound represented by the above formula (1) which is monoesterified or diesterified by medium-chain fatty acids. Preferred medium-chain fatty acids are fatty acids containing 8 to 12 carbon atoms, and they are straight chain saturated fatty acids having an even number of carbon atoms, that is, caprylic acid (octanoic acid), capric acid (decanoic acid), and lauric acid (dodecanoic acid).

Further, the term, long chain fatty acid, is used in the present specification to mean a fatty acid containing more carbon atoms than the medium-chain fatty acid. This is to say, it means a fatty acid containing 14 or more carbon atoms.

Synthesis of astaxanthin medium-chain fatty acid ester

The present inventors have found by the above described experiments that an astaxanthin medium-chain fatty acid ester has better intestinal absorption rate and accumulate in liver tissue much better than those of a long chain fatty acid ester. Taking into consideration the usefulness of the medium-chain fatty acid ester form astaxanthin and the application in the field of food, the present inventors have made intensive studies regarding a method of synthesizing an astaxanthin medium-chain fatty acid ester using lipase, and as a result, they have completed the present invention. The synthesis method of the present invention includes the following embodiments:

(1) Esterification

One embodiment of the present invention is a method of producing an astaxanthin medium-chain fatty acid monoester or astaxanthin medium-chain fatty acid diester, which is characterized in that a medium-chain fatty acid is used as a fatty acid when esterification is carried out between astaxanthins and fatty acids using lipase. The medium-chain fatty acid is a straight chain saturated fatty acid containing 8 to 12 carbon atoms.

It is known that generally esterification with lipase is carried out while eliminating as much water as possible. In the method disclosed in Japanese Patent Laid-Open (JPA) No. 11-290094, an astaxanthin ester is synthesized by adding a very small amount of water (0.02 to 0.1%). However, in the method of the prior art technique, if a medium-chain fatty acid is used instead of a long chain fatty acid, esterification is not sufficiently carried out. In the present invention, as shown in Table 1, several percent of water is added to a reaction system to solve this problem. Thus, surprisingly, esters are formed from astaxanthins and medium-chain fatty acids.

(Table 1) Water added to reaction and ester-generating activity

| | Dried enzymes | | Immobilized enzyme | |
|---|---|---|---|---|
| Water(%) | Octanoic acid | MCT | Octanoic acid | MCT |
| 0 | nd | nd | nd | nd |
| 1 | 4.9 | nd | nd | nd |
| 2 | 6.0 | nd | 1.4 | 1.0 |
| 5 | 3.5 | 3.4 | 4.0 | 11.4 |
| 7.5 | 4.0 | 3.5 | 4.1 | 16.5 |
| 10 | 3.4 | 3.3 | 3.4 | 16.5 |
| 15 | 3.1 | 2.0 | 1.7 | 10.5 |

(Octanoic-monoester-generating rate (%))
nd: not detected

(2) Transesterification with medium-chain fatty acid triglyceride

The above described remarkable effect obtained by the addition of water is also found in transesterification using lipase. That is to say, even if a medium-chain fatty acid triglyceride (MCT) is used as a substrate providing a fatty acid (fatty acid donor) instead of a free fatty acid, transesterification progresses by increasing the additive amount of water, and an astaxanthin medium-chain fatty acid ester is obtained. The amount of water added to a reaction system in this embodiment will be described in detail later.

Esterification can be carried out using an alkyl ester of medium-chain fatty acid as well as a medium-chain fatty acid triglyceride, diglyceride and monoglyceride. Preferred examples of such an alcohol ester include lower alcohol esters (e.g., methanol, ethanol, n-propanol, n-butanol).

Esterification can also be carried out, using lipase, which is immobilized on ion exchange resin or the like for stabilization. Such immobilized enzyme can be used, also when a free or alkyl ester form medium-chain fatty acid is used for transesterification. The esterification of a medium-chain fatty acid triglyceride by lipase has the yield of an astaxanthin medium-chain fatty acid ester of interest much higher than that of the esterification of a free medium-chain fatty acid.

(3) Transesterification with other astaxanthin esters

The method of the prior art technique (Japanese Patent Laid-Open (JPA) No. 11-290094) is limited to the esterification of astaxanthins having free OH residues (the method disclosed in Japanese Patent Laid-Open (JPA) No.

11-290094 is a reaction that is the esterification from monoester form to diester form astaxanthin, but not transesterification between monoester form of astaxanthin and a free or triglyceride form of fatty acid.).

In contrast, according to the method of the present invention, as described in detail later, the additive amount of water and the conditions for enzyme reaction are determined as appropriate, whereby the fatty acid portion of a long chain fatty acid monoester or diester form astaxanthin can be converted into a medium-chain fatty acid by transesterification. For example, a chemically prepared astaxanthin oleic acid diester can be converted to an astaxanthin octanoic acid monoester (astaxanthin medium-chain fatty acid ester).

In the case of transesterification with other astaxanthin esters also, dried lipase may be used, or it may preferably be immobilized and used. Moreover, the medium-chain fatty acid may be used as a free fatty acid, or more preferably as triglyceride. Otherwise, it may also be used as an alcohol ester other than triglyceride.

As stated above, the method of the present invention has a practical advantage, when the method is carried out in transesterification reaction. This is because the astaxanthin that is obtained currently most easily for the use as an astaxanthin source is an astaxanthin prepared from the culture of Chlorophyceae, or extracted from Euphausiacea, and because these astaxanthins contain a mixture of different kinds of long chain fatty acid esters (the mixture of a monoester and a diester). When such astaxanthins are used as reaction materials, as long as the method of Japanese Patent Laid-Open (JPA) No. 11-290094 is applied, astaxanthin diester having both long-chain and medium-chain fatty acids can only be obtained, and further, the diester form contained in the materials remains unchanged. In contrast, according to the method of the present invention, water is effectively added as described above, and thereby astaxanthin medium-chain fatty acid esters can be obtained from astaxanthins extracted from the nature and medium-chain fatty acids or triglycerides thereof by transesterification using lipase.

The present invention provides a composition comprising 0.1% or more of the astaxanthin medium-chain fatty acid monoester or astaxanthin medium-chain fatty acid diester synthesized using the above lipase.

The present invention provides a method of producing by fatty acid ester transesterification by lipase, an astaxanthin medium-chain fatty acid monoester and an astaxanthin medium-chain fatty acid diester, which can be expected to be applied in the field of cosmetics or food, and compositions comprising these astaxanthins.

Moreover, the present invention further provides a method of producing an astaxanthin medium-chain fatty acid monoester and an astaxanthin medium-chain fatty acid diester, preferably the natural product of an astaxanthin octanoic acid monoester or astaxanthin octanoic acid diester, by extraction preferably from Crustacea, and more preferably from Euphausiacea.

The present invention will be described in detail below.

The production of a composition comprising an astaxanthin medium-chain fatty acid monoester and an astaxanthin medium-chain fatty acid diester by enzyme method is carried out as follows.

<u>Enzyme</u>

The present inventors have made studies using commercially available enzymes. As a result, they have found that enzymes used in esterification or transesterification of astaxanthin include lipase derived from <u>Candida</u> [e.g., lipase derived from <u>Candida rugosa</u> (Meito Sangyo Co., Ltd., product name: Lipase OF, etc.), lipase derived from <u>Candida rugosa</u> (Meito Sangyo Co., Ltd., product name: Lipase MY, etc.), lipase derived from <u>Candida rugosa</u> (Amano Enzyme Inc., product name: Lipase AY "Amano" 30G, etc.), lipase derived from <u>Candida antarctica</u> (Novo Industry, product name: Novozym435, etc.)], lipase derived from microorganisms of <u>Chromobacterium</u> [e.g., lipase derived from <u>Chromobacterium viscosum</u>, Asahi Kasei Corporation, product name: Lipase AC, etc.], lipase derived from microorganisms of <u>Alcaligenes</u> [e.g., lipase derived from <u>Alcaligenes</u> sp. (Meito Sangyo Co., Ltd., product name: Lipase PL, etc.)], and lipase derived from the pancreas of animals. However, lipase used in the present invention is not limited thereto. Any lipase may be used, as long as it acts on a solution containing the OH group of an astaxanthin or esters thereof and the medium-chain fatty acid, and it can synthesizes an astaxanthin medium-chain fatty acid monoester or astaxanthin medium-chain fatty acid diester by the transesterification of fatty acids. The origin or type of lipase is not limited. Lipase derived from <u>Candida</u> is preferable in terms of yield. In order to enhance enzymatic activity, suppress the denaturation of materials, or increase reaction yield, these lipases may be purified before use. Examples of enzyme purification include drying, salting-out, and column chromatography.

Lipase used in the present invention can be dissolved or dispersed in an aqueous solvent, or dried lipase can be used as is. Also, it can be immobilized on a carrier and used as immobilized lipase. If lipase is used as immobilized lipase, the enzyme is stabilized, and it can be recycled, thereby reducing production cost. The immobilization of lipase is carried out by a known method. As an immobilizing carrier, known carriers such as silica gel, celite, κ-carrageenan, chitin or sodium alginate can be used [Bioreactor, edited by Saburo Fukui, Kodansha Scientific (1985); Jissen Bioreactor, edited by The Japanese Research & Development Association for Bioreactor System in Food Industry, Food Chemical News (1990)]. Moreover, lipase can be immobilized on ion exchange resin which is used in purification of water. Furthermore, lipase can also be immobilized on resin used in adsorption chromatography or hydrophobic adsorption chromatography. Generally, lipase can be immobilized on a resin carrier capable of adsorbing a protein.

Reaction material, astaxanthin

An astaxanthin used as a material for esterification may be either a free astaxanthin or an astaxanthin fatty acid ester, and it may also use only a free form or estesifiedform of astaxanthin, and use or mixture of a free form or estesified form of astaxanthin. The free astaxanthin may be either a synthetic product (a commercially available product from Roche, Sigma) or an extract from the nature. Further, an astaxanthin obtained by culturing Phaffia yeast, accumulating it in cell bodies, and extracting or purifying it therefrom, may also be used. Furthermore, an astaxanthin obtained by culturing or breeding microorganisms, yeast, Fungi or plants that are bred by genetic engineering, and extracting or purifying it from these, may also be used. The fatty acid ester of an astaxanthin may be a monoester, diester, or a mixture of both esters. Still further, the astaxanthin fatty acid ester can be either a synthetic product or natural extract. For example, an astaxanthin fatty acid ester extracted from the nature such as Haematococcus from Chlorophyceae, Crustacea such as Euphausiacea, shrimps or crabs, or the eggs of fish is the mixture of a monoester form and a diester form, and an esterified fatty acid is also the mixture of various fatty acids. However, these types of fatty acid esters of astaxanthin can be used without any problem. Still further, two or more types of the above described free astaxanthins and astaxanthin fatty acid esters can be used in combination.

Reaction material, fatty acids

As a medium-chain fatty acid that is the substrate used in esterification, a straight chain saturated fatty acid containing 8 to 12 carbon atoms is desired. Specific examples include a fatty acid selected from a group consisting of caprylic acid, capric acid and lauric acid, and a mixed fatty acid consisting of two or more selected from the above group. Moreover, in the present invention, triglyceride or fatty acids having other ester forms, which have higher reactivity than the free fatty acid, can also be used. Specific examples include a straight chain triglyceride fatty acid containing 8 to 12 carbon atoms and an alcohol ester thereof.

Reaction temperature

Transesterification and esterification using an enzyme is generally carried out under conditions of a reaction temperature of 20°C to 55°C. The present enzyme reaction is desirably carried out under conditions in which the optimal temperature and the optimal pH of each lipase are used. However, if the reaction temperature is higher than 50°C, a reaction substrate astaxanthin is increasingly decomposed or isomerized, and so it is not preferable. In contrast, if the reaction temperature is lower than 20°C, lipase activity decreases and lipid as a substrate (fatty acid, triglyceride, etc.) becomes a solid, and so it is also not preferable. Taking into consideration these points, enzyme reaction is more preferably carried out at a temperature of 37°C to 50°C. In the present invention, in order to prevent the decomposition of the astaxanthin due to oxidization, it is also preferable to carry out the enzyme reaction in an inert gas atmosphere such as nitrogen or argon gas.

Amount of lipase used in reaction

The amount of lipase used in the present invention is 100 u (= unit)/μmol to 30000 u/μmol, and preferably 1000 u/μmol to 30000 u/μmol with respect to the amount of an astaxanthin. If the amount of lipase is less than 100 u/μmol, an astaxanthin fatty acid ester cannot be obtained at a high yield. When a free astaxanthin is used as a substrate, even if more than 50000 u/μmol of lipase is used, a remarkable effect cannot be expected, and so it is not preferable. On the other hand, when a mixture of a free and ester form of astaxanthin is used as a substrate, the amount of the synthesized product is increased, as the amount of the used enzyme is increased. However, taking into consideration the loading dose of the enzyme to an immobilizing carrier or the cost efficiency regarding the amount of enzyme used, the above described amount is considered to be adequate.

Ratio between astaxanthin and fatty acid in reaction

The molar ratio between the astaxanthin and the fatty acids used in the present invention is broadly divided into several categories, depending on the number of fatty acid groups of the oil and fats used. That is to say, it can broadly be divided into a case where fatty acids consist of a free medium-chain fatty acid and an alcohol ester form medium-chain fatty acid, and a case where three molecules of fatty acids are bound to make a medium-chain triglyceride. In the case of the former, the fatty acids are present at a molar ratio of 30 to 10,000, and preferably 30 to 3,500 with respect to astaxanthin based on a conversion ratio of free astaxanthin. In the case of the latter, the fatty acids are present

at a molar ratio of 10 to 3,000, and preferably 30 to 1,000 with respect to astaxanthin based on a conversion ratio of free astaxanthin.

When astaxanthins have a concentration higher than the above magnification, astaxanthin is not sufficiently dissolved, and accordingly it is not adequate for reaction. When astaxanthins have a concentration lower than the above magnification, significant improvement of effects cannot be expected, and transesterification does not progress due to the rarefaction of astaxanthin concentration.

Reaction time

The reaction time for the enzyme reaction in the present invention is desirably 12 hours or longer. If the reaction time is short, the reaction does not progress very much, and so it is not preferable. Since the enzyme reaction progresses relatively slowly and the decomposition of astaxanthin esters during the reaction also progresses relatively slowly, it is desirable to set the reaction time rather longer to increase the generation yield of a product of interest. In the present invention, in order to prevent the decomposition of the astaxanthin due to oxidization, it is also preferable to carry out the enzyme reaction in an inert gas atmosphere such as nitrogen or argon gas.

Organic solvent used in reaction and the amount

In the enzyme reaction in the present invention, an organic solvent can be used during the reaction. Considering the stability of lipase during the reaction, a nonpolar solvent is preferable. Specific examples of such a nonpolar solvent include n-hexane, benzene, carbon tetrachloride, acetone and others. Any of these solvents can be used, but considering the application to food or the like, h-hexane is more preferable in terms of toxicity and safety. The solution of the astaxanthin or fatty acid esters (substrate) has a high viscosity. If the substrate solution is diluted with an organic solvent such as n-hexane, the viscosity of the solution can be decreased, thereby enabling efficient reaction. In particular, when lauric acid or lauric acid triglyceride, which has a high melting point and becomes a solid at an ordinary reaction temperature, is used, the use of an organic solvent is effective. The organic solvent used in the present invention is desirably used at an amount of 1000 or less times, and more preferably 200 or less times of reactive oils and fats (a total of astaxanthin and fatty acids). Even if the organic solvent is used at an amount higher than the above amount, the reaction is not promoted, and much effort is expended to remove hexane after completion of the reaction. Accordingly, it is not advisable to do so.

Additive amount of water in lipase reaction

Lipase reaction is a reversible reaction. If the amount of water is large, the generated astaxanthin medium-chain fatty acid esters are hydrolyzed. Accordingly, it has been common sense to reduce the amount of water to the minimum in such synthetic reaction or transesterification. However, as stated above, as a result of intensive studies by the present inventors, it was found that if water is positively added in a certain range, the progression of esterification or transesterification is promoted. The additive amount of water is desirably at a ratio of 0.5 to 20% to reactive oils and fats (astaxanthin and fatty acids). More preferably, the ratio of 2 to 15% is desired. Even if the content of water exceeds 20%, reaction progresses. However, the decomposition of the generated esters also progresses, and the generation yield of astaxanthin medium-chain esters is reduced. Therefore, it is not preferable.

Purification after completion of reaction

Examples of a method of producing from the enzyme reaction solution of the present invention, an astaxanthin medium-chain fatty acid monoester or astaxanthin medium-chain fatty acid diester, which is made by the covalent binding of one or two molecules of medium-chain fatty acid residues to an astaxanthin of interest, include common methods of purifying oils and fats such as degumming, deacidification or steam distillation, vacuum precision distillations such as molecular distillation, purification method by chromatography such as silica gel chromatography, and the combined use of these methods. According to these methods, it becomes possible to eliminate a long chain fatty acid bound to a substrate astaxanthin ester form that is cleaved during ester transition, a free medium-chain fatty acid cleaved from a medium-chain fatty acid triglyceride, and an excessive reactive substrate that is a medium-chain fatty acid triglyceride or free medium-chain fatty acid.

Production of astaxanthin medium-chain fatty acid monoester and astaxanthin medium-chain fatty acid diester by extraction from natural products

A natural product used as a material includes preferably Crustacea, and particularly preferably Euphausiacea.

As a Euphausiacea, a commercially available Euphausiacea can be used. As a solvent used in extraction, any solvent can be used, as long as the extraction of an astaxanthin medium-chain fatty acid monoester and an astaxanthin medium-chain fatty acid diester can be carried out with the solvent. A preferred solvent is acetone. In the extraction step, not only organic solvents but supercritical $CO_2$ can also be used. The purification of the astaxanthin medium-chain fatty acid monoester and the astaxanthin medium-chain fatty acid diester can be carried out, for example, by silica column chromatography or ODS column chromatography.

Food comprising composition containing astaxanthin medium-chain fatty acid monoester and astaxanthin medium chain fatty acid diester obtained by enzyme method

Use as food materials

Oils and fats containing the astaxanthin medium-chain fatty acid ester of the present invention have unlimited possibilities for use. As a possible use, the oils and fats can be used as food materials and additives.

Astaxanthin has the highest red color property among carotenoids and is relatively stable to heat, light, pH and the like. Accordingly, a Phaffia pigment (a free astaxanthin) and a Haematococcus pigment (a long chain fatty acid ester form astaxanthin) have been used as natural pigment(food additives). Moreover, since the astaxanthin shows strong antioxidant activity, it receives attention as a new functional, natural pigment that has never existed before. A medium-chain fatty acid was enzymatically esterified to such an astaxanthin having useful functions, and as a result, oils and fats containing the astaxanthin medium-chain fatty acid ester having excellent digestibility could be produced.

The red color tone of the astaxanthin medium-chain fatty acid ester is the same as that of astaxanthins contained in the conventional Phaffia and Haematococcus pigment. Thus, as with the Phaffia and Haematococcus pigment, the astaxanthin medium-chain fatty acid ester-containing oils and fats can be added to food. The astaxanthin medium-chain fatty acid ester-containing oils and fats having good digestibility as well as antioxidant activity are expected to function in food better than the conventional Phaffia pigment and the like.

Actually, the astaxanthin medium-chain fatty acid ester-containing oils and fats can be added to beverages such as juice, alcoholic beverages such as liquor, confectionery, fishery products such as fish sausage, and condiments such as dressing or ketchup.

The above astaxanthin medium-chain fatty acid ester-containing oils and fats can be used in the form of an emulsified product or powder. The emulsified product can be produced by adding the following food emulsifiers to the astaxanthin medium-chain fatty acid ester-containing oils and fats and stirring the mixture. Examples of a food emulsifier permitted under the Food Sanitation Law include nonionic activators such as fatty acid monoglycerides, polyglycerin fatty acids, sorbitan fatty acid esters (span, etc.) and sucrose fatty acid ester, and natural products such as lecithin, enzymatically treated lecithin, gum Arabic, Quillaja saponaria extract and egg yolk.

Use as healthy food and/or supplements materials

Oils and fats containing the astaxanthin medium-chain fatty acid ester of the present invention have unlimited possibilities for use. The oils and fats can also be used as healthy food and/or supplements materials and additives. The base structure of the main ingredient of the astaxanthin medium-chain fatty acid ester-containing oils and fats is astaxanthin. As stated in the above Use as food materials, astaxanthin receives attention as a functional pigment.

For the use, as in the case of food materials, the oils and fats may be emulsified or powdered for addition. In the present invention, depending on the healthy food and/or supplements to which the oils and fats are added, an optimal food emulsifier is selected, and it is used in the optimal amount. An emulsified product can be produced by adding the following food emulsifiers to the astaxanthin medium-chain fatty acid ester-containing oils and fats, and stirring the mixture. Examples of a food emulsifier permitted under the Food Sanitation Law include nonionic activators such as fatty acid monoglycerides, polyglycerin fatty acids, sorbitan fatty acid esters (span, etc.) and sucrose fatty acid ester, and natural products such as lecithin, enzymically treated lecithin, gum Arabic, Quillaja saponaria extract and egg yolk.

When the astaxanthin medium-chain fatty acid ester-containing oils and fats are encapsulated, examples of a protein used as a coating agent for the oils and fats and as an emulsion stabilizer include plant proteins such as soy beans or corns, animal proteins such as skimmed milk, ovalbumin, casein, whey or gelatin, and others. Similarly, examples of carbohydrate used in the encapsulation include starch from corn, tapioca, sweet potato or potato, carrot powder, dextrin, sucrose, glucose, lactose and others. In addition, as an emulsion stabilizer, salts such as potassium phosphate, sodium phosphate or sodium citrate, natural gums such as gum Arabic or pectin, sodium alginate, and others can also be used. To prevent oxidation, antioxidants such as tocopherol can also be used.

Examples

The present invention is further specifically described in the following production examples and examples. Needless to say, these examples are provided for illustrative purposes only, and are not intended to limit the scope of the invention.

Determination of lipase activity

Five ml of an olive oil emulsion (obtained by placing 22.9 g of an olive oil and 75 ml of a polyvinyl alcohol solution in an emulsifying device, and emulsifying the mixture at 5°C to 10°C) and 4 ml of a 0.1 M phosphate buffer (pH 7.0) were placed in a 50 ml-volume Erlenmeyer flask with a ground-in stopper followed by fully mixing. Then, the mixed solution was placed in a bath with a constant temperature of 37°C, and it was preheated for 10 minutes. While stirring, 1 ml of an enzyme solution was added to this solution followed by reaction for 20 minutes. Twenty ml of an acetone-ethanol mixed solution (obtained by mixing acetone and ethanol at a ratio of 1 : 1 (V/V)) was added to the solution to terminate the reaction. Several drops of phenolphthalein solution were added thereto, and titration was carried out with a 0.05 N sodium hydroxide solution so as to determine the amount of a free fatty acid generated as a result of lipase reaction.

A blank test was carried out by using only the above olive oil emulsion and phosphate buffer, adding the acetone-ethanol mixed solution, then adding the enzyme solution followed by titration (the titration value of a control solution).

Enzyme activity is calculated by the following formula:

$$\text{Enzyme activity (unit/g)} = (\text{titer value of sample solution} - \text{titer value of control solution})/(\text{enzyme g in 1 ml of enzyme solution}) \times 2.5$$

Chemical synthesis of astaxanthin medium-chain fatty acid ester

Production example 1 Chemical synthesis of astaxanthin octanoic acid diester

Octanoic acid (0.73 g, 5.06 mmol) and 4-(dimetylamino)pyridine (DMAP) (218.7 mg, 1.79 mmol) were added to a dry methylene chloride solution (20 ml) comprising an astaxanthin (995.2 mg, 1.67 mmol) and 1-[3-(dimethylamino) propyl]-3-ethyl carbodiimide hydrochloride (WSC-HCl) (1.28 g, 6.68 mmol) under argon at room temperature.

After 19 hours, the reaction mixture was poured into ethyl acetate (200 ml), and then the mixture was washed with 1 M hydrochloric acid (100 ml), a saturated sodium bicarbonate solution (100 ml) and a saturated saline solution (100 ml) successively.

The organic phase was dried with anhydrous sodium sulfate, and the solvent was removed under a reduced pressure. Thereafter, it was dissolved in 10 ml of methylene chloride-hexane (1 : 1, V/V), and column chromatography was carried out using silica gel (175 g). A dark red solid (1.27 g, 90%) of astaxanthin octanoic acid diester was obtained from ethyl acetate-hexane (1 : 2, V/V) by elution with the ethyl acetate-hexane.

Astaxanthin octanoic acid diester

$^1$H-NMR: δ(TMS) 0.88(t, 6H, J=7Hz), 1.20-1.45(m, 16H), 1.22(s, 6H), 1.35(s, 6H), 1.65-1.75(m, 4H), 1.90(s, 6H), 1.95-2.1 (m, 4H), 1.99(s, 6H), 2.00(s, 6H), 2.35-2.55(m, 4H), 5.53(dd, 2H, J=6Hz, 14Hz), 6.15-6.7(m, 14H).

Production example 2 Chemical synthesis of astaxanthin octanoic acid monoester

Octanoic acid (0.27 g, 1.87 mmol) and DMAP (107.5 mg, 0.88 mmol) were added to a dry methylene chloride solution (20 ml) comprising an astaxanthin (976.2 mg, 1.64 mmol) and WSC·HCl (0.48 g, 2.50 mmol) under argon at room temperature.

After 18 hours, the reaction mixture was poured into methylene chloride (250 ml), and then the mixture was washed with 1 M hydrochloric acid (100 ml), a saturated sodium bicarbonate solution (100 ml) and a saturated saline solution (100 ml) successively.

The organic phase was dried with anhydrous sodium sulfate, and the solvent was removed under a reduced pressure. Thereafter, it was dissolved in 15 ml of methylene chloride-hexane (2 : 1, V/V), and column chromatography was carried out using silica gel (250 g). By elution with ethyl acetate-hexane, a dark red solid (304.2 mg, 22%) of

astaxanthin octanoic acid diester was obtained from ethyl acetate-hexane (1 : 2, V/V), and a dark red solid (377.3 mg, 32%) of astaxanthin octanoic acid monoester was obtained from the ethyl acetate-hexane (1 : 1, V/V).
Astaxanthin octanoic acid monoester
$^1$H-NMR: δ(TMS) 0.89(t, 3H, J=7Hz), 1.20-1.45(m, 8H), 1.21(s, 3H), 1.22(s, 3H), 1.32(s, 3H), 1.35(s, 3H), 1.65-1.75(m, 2H), 1.81(dd, 1H, J=13Hz, 13Hz), 1.90(s, 3H), 1.94(s, 3H), 1.95-2.1(m, 2H), 1.99(s, 6H), 2.00(s, 6H), 2.15(dd, 1H, J=6Hz, 13Hz), 2.35-2.55(m, 2H), 3.67(d, 1H, J=2Hz), 4.32(ddd, 1H, J=2Hz, 6Hz, 13Hz), 5.53(dd, 1H, J=6Hz, 14Hz), 6.15-6.75 (m, 14H).

Production example 3 Astaxanthin decanoic acid diester, astaxanthin decanoic acid monoester

In the same manner as in Production example 2, a dark red solid (17.5 g, 56%) of astaxanthin decanoic acid diester and a dark red solid (9.6 g, 37%) of astaxanthin decanoic acid monoester were obtained from astaxanthin (20.6 g, 34.5 mmol), WSC·HCl (14.9 g, 77.7 mmol), decanoic acid (9.8 g, 56.9 mmol) and DMAP (2.8 g, 22.9 mmol). Astaxanthin decanoic acid diester
$^1$H-NMR($CDCl_3$): δ(TMS) 0.88(t, 6H, J=7Hz), 1.20-1.45(m, 24H), 1.22(s, 6H), 1.35(s, 6H), 1.65-1.75(m, 4H), 1.90(s, 6H), 1.95-2.1(m, 4H), 1.99(s, 6H), 2.00(s, 6H), 2.35-2.55(m, 4H), 5.53(dd, 2H, J=6Hz, 14Hz), 6.15-6.7(m, 14H).
Astaxanthin decanoic acid monoester
$^1$H-NMR($CDCl_3$): δ(TMS) 0.88(t, 3H, J=7Hz), 1.20-1.45(m, 12H), 1.21(s, 3H), 1.22(s, 3H), 1.32(s, 3H), 1.35(s, 3H), 1.65-1.75(m, 2H), 1.81(dd, 1H, J=13Hz, 13Hz), 1.90(s, 3H), 1.95(s, 3H), 1.95-2.1(m, 2H), 1.99(s, 6H), 2.00(s, 6H), 2.15 (dd, 1H, J=6Hz, 13Hz), 2.35-2.55(m, 2H), 3.69(bs, 1H), 4.32(dd, 1H, J=6Hz, 14Hz), 5.53(dd, 1H, J=6Hz, 14Hz), 6.15-6.75 (m, 14H).

Chemical synthesis of astaxanthin long chain fatty acid ester

Production example 4 Astaxanthin palmitin acid diester

In the same manner as in Production example 1, a dark red solid (582.9 mg, 93%) of astaxanthin palmitin acid diester was obtained from astaxanthin (348.3 mg, 0.584 mmol), WSC·HCl (0.46 g, 2.40 mmol), palmitin acid (0.46 g, 1.79 mmol) and DMAP (57.0 mg, 0.467 mmol).
Astaxanthin palmitin acid diester
$^1$H-NMR: δ(TMS) 0.89(t, 6H, J=7Hz), 1.20-1.45(m, 48H), 1.23(s, 6H), 1.35(s, 6H), 1.65-1.75(m, 4H), 1.91(s, 6H), 1.95-2.10 (m, 4H), 1.99(s, 6H), 2.00(s, 6H), 2.35-2.55(m, 4H), 5.54(dd, 2H, J=6Hz, 14Hz), 6.15-6.7(m, 14H).

Production example 5 Astaxanthin oleic acid diester

In the same manner as in Production example 1, a dark red oily product (615.6 mg, 87%) of astaxanthin oleic acid diester was obtained from astaxanthin (376.1 mg, 0.630 mmol), WSC·HCl (0.49 g, 2.56 mmol), oleic acid (0.53 g, 1.89 mmol) and DMAP (58.2 mg, 0.476 mmol).
Astaxanthin oleic acid diester
$^1$H-NMR: δ(TMS) 0.88(t, 6H, J=7Hz), 1.15-1.45(m, 40H), 1.23(s, 6H), 1.35(s, 6H), 1.65-1.80(m, 4H), 1.90(s, 6H), 1.95-2.15 (m, 12H), 1.99(s, 6H), 2.00(s, 6H), 2.35-2.55(m, 4H), 5.30-5.45(m, 4H), 5.54(dd, 2H, J=6Hz, 14Hz), 6.15-6.7(m, 14H).

Immobilization of lipase

Several types of lipase derived from yeast are on the market, and an example of such lipase includes Lipase OF (product name) derived from Candida, which can be purchased from Meito Sangyo Co., Ltd. Examples of the immobilization of the Lipase OF on ion exchange resin or the like will be explained below.

"Immobilization on ion exchange resin (direct method)"

One hundred gram (wet weight) of an ion exchange resin carrier (Dowex MARATHON WBA: Dow Chemical) was suspended in 80 ml (5,760,000 units) of a Candida rugosa lipase solution (Lipase OF bulk, 12.5%: Meito Sangyo Co., Ltd.), and the suspension was dried under a reduced pressure to obtain an immobilized enzyme (71.0 g).

"Immobilization on ion exchange resin (dialysis method)"

Sixteen gram (5,760,000 units) of a Candida rugosa lipase (powder, Meito Sangyo Co., Ltd., product name: Lipase OF) was dissolved in 80 ml of distilled water, and the suspension was dialyzed overnight with 5 L of distilled

water. After completion of the dialysis, insoluble matters were eliminated using a centrifuge to obtain a clear lipase solution. One hundred gram (wet weight) of an ion exchange resin carrier (Dowex MARATHON WBA: Dow Chemical) was suspended in the obtained lipase solution, and the suspension was dried under a reduced pressure to obtain an immobilized enzyme (62.8 g).

"Immobilization on ion exchange resin (column method)"

In order to immobilize a lipase on ion exchange resin more efficiently than by the above described methods, immobilized enzyme was obtained by the following immobilization method.

Thirty-two gram (11,520,000 units) of a Candida rugosa lipase (powder, Meito Sangyo Co., Ltd., product name: Lipase OF) was dissolved in 160 ml of distilled water, and the mixture was dialyzed overnight with 10 L of distilled water. After completion of the dialysis, insoluble matters were eliminated using a centrifuge to obtain a clear lipase solution. One hundred gram (wet weight) of an ion exchange resin carrier (Dowex MARATHON WBA: Dow Chemical) was filled in a column, and the lipase solution was supplied thereto at a flow rate of 1 ml/min so as to adsorb the lipase on the ion exchange resin carrier. The adsorption carrier was dried under a reduced pressure to obtain an immobilized enzyme (66.7 g).

"Immobilization on hydrophobic adsorptive resin, adsorptive resin or filtration auxiliary agent"

(Phenyl)

One hundred ml of a hydrophobic adsorptive resin carrier (Phenyl Toyopearl 650S: Tosoh Corporation) was suspended in 80 ml (5,760,000 units) of a Candida rugosa lipase dialyzed solution (Lipase OF bulk, 12.5%: Meito Sangyo Co., Ltd.), and the suspension was dried under a reduced pressure to obtain an immobilized enzyme (24.9 g).

(HP20)

One hundred gram (wet weight) of an aromatic adsorptive resin carrier (DIAION HP20: Mitsubishi Chemical Corporation) was suspended in 80 ml (5,760,000 units) of a Candida rugosa lipase solution (Lipase OF bulk, 12.5%: Meito Sangyo Co., Ltd.), and the suspension was dried under a reduced pressure to obtain an immobilized enzyme (60.3 g).

(HP1MG)

Twenty-five gram (wet weight) of a methacryl adsorptive resin carrier (DIAION HP1MG: Mitsubishi Chemical Corporation) was suspended in 80 ml (5,760,000 units) of a Candida rugosa lipase solution (Lipase OF bulk, 12.5%: Meito Sangyo Co., Ltd.), and the suspension was dried under a reduced pressure to obtain an immobilized enzyme (13.1 g).

(HPA25)

Twenty-five gram (wet weight) of a high porous-type aromatic adsorptive ion exchange resin carrier (DIAION HPA25: Mitsubishi Chemical Corporation) was suspended in 80 ml (5,760,000 units) of a Candida rugosa lipase solution (Lipase OF bulk, 12.5%: Meito Sangyo Co., Ltd.), and the suspension was dried under a reduced pressure to obtain an immobilized enzyme (12.3 g).

(Filtration auxiliary agent: celite)

Forty-three gram of celite (Hyflo super-cel: Nacalai Tesque, Inc.) was suspended in 80 ml (5,760,000 units) of a Candida rugosa lipase dialyzed solution (Lipase OF bulk, 12.5%: Meito Sangyo Co., Ltd.), and the suspension was dried under a reduced pressure to obtain an immobilized enzyme (45.5 g).

"Immobilization of lipase derived from microorganisms"

A large number of lipases derived from organisms other than yeast are present. An example is a lipase derived from bacteria belonging to Alcaligenes genus, and this lipase can be purchased from Maito Sangyo Co., Ltd. for use. The example of immobilizing this lipase is also described below.

Sixteen gram (1,440,000 units) of an Alcaligenes lipase (powder, Meito Sangyo Co., Ltd., product name: Lipase

PL) was dissolved in 80 ml of distilled water, and the suspension was dialyzed overnight with 5 L of distilled water. After completion of the dialysis, insoluble matters were eliminated using a centrifuge to obtain a clear lipase solution. One hundred gram (wet weight) of an ion exchange resin carrier (Dowex MARATHON WBA: Dow Chemical) was suspended in the obtained lipase solution, and the suspension was dried under a reduced pressure to obtain an immobilized enzyme (64.0 g).

Examples of enzyme reaction

Example 1

Two mg of a free astaxanthin (manufactured by Sigma) and 300 mg of octanoic acid as a free fatty acid were placed in a brown glass bottle. Eighty mg of lipase derived from Candida (manufactured by Meito Sangyo Co., Ltd., product name: Lipase OF) was added thereto, and 30 μL of water was further added thereto and stirred fully afterwards. Thereafter, while stirring, reaction was carried out at 45°C. Three days later, the reaction solution was taken out, and the composition ratio of astaxanthin was analyzed by HPLC. As a result, the composition ratio was 94.0% astaxanthin, 6.0% monoester, and less than 1% diester.

When an astaxanthin extracted from Phaffia yeast was used instead of the free astaxanthin manufactured by Sigma, almost the same results were obtained. The composition ratio in that case was 93.5% astaxanthin, 6.5% monoester, and less than 1% diester.

Example 2

Two mg of a free astaxanthin (manufactured by Sigma) and 300 mg of tricaprilin as a triglyceride form fatty acid were placed in a brown glass bottle. One hundred twenty mg of lipase derived from Candida (manufactured by Meito Sangyo Co., Ltd., product name: Lipase OF) was added thereto, and 30 μL of water was further added thereto and stirred fully afterwards. Thereafter, while stirring, reaction was carried out at 45°C. Three days later, the reaction solution was taken out, and the composition ratio of astaxanthin was analyzed by HPLC. As a result, the composition ratio was 84.9% astaxanthin, 13.6% monoester, and 1.5% diester.

Example 3

Two mg of the astaxanthin oleic acid diester described in Production example 5 and 300 mg of octanoic acid as a free fatty acid were placed in a brown glass bottle. Eighty mg of lipase derived from Candida (manufactured by Meito Sangyo Co., Ltd., product name: Lipase OF) was added thereto, and 30 μL of water was further added thereto and stirred fully afterwards. Thereafter, while stirring, reaction was carried out at 45°C. Three days later, the reaction solution was taken out, and the composition ratio of astaxanthin was analyzed by HPLC. As a result, the composition ratio was 61.1% astaxanthin, 8.2% monoester, and less than 1% diester.

Example 4

Two mg of the astaxanthin oleic acid diester described in Production example 5 and 300 mg of tricaprilin as a triglyceride form fatty acid were placed in a brown glass bottle. Eighty mg of lipase derived from Candida (manufactured by Meito Sangyo Co., Ltd., product name: Lipase OF) was added thereto, and 30 μL of water was further added thereto and stirred fully afterwards. Thereafter, while stirring, reaction was carried out at 45°C. Three days later, the reaction solution was taken out, and the composition ratio of astaxanthin was analyzed by HPLC. As a result, the composition ratio was 73.4% astaxanthin, 15.3% monoester, and less than 1% diester. The astaxanthin oleic acid diester that was not converted but remained was 7.9%. In order to confirm whether the remaining astaxanthin oleic acid diester completely disappears in the enzyme reaction, the present reaction was carried out for 1 week, and then the composition ratio of astaxanthin was analyzed by HPLC. As a result, the composition ratio was 72.8% astaxanthin, 27.2% monoester, and less than 1% diester, and the astaxanthin oleic acid diester as a substrate was not detected.

Example 5

Twenty-four mg of a mixture of astaxanthin esters extracted from the nature (manufactured by Itano, product name: Astax9000H) and 276 mg of octanoic acid as a free fatty acid were placed in a brown glass bottle. Two hundred and seventy mg of lipase derived from Candida (manufactured by Meito Sangyo Co., Ltd., product name: Lipase OF) was added thereto, and 22.5 μL of water was further added thereto and stirred fully afterwards. Thereafter, while stirring, reaction was carried out at 45°C. Three days later, the reaction solution was taken out, and the composition ratio of

astaxanthin was analyzed by HPLC. As a result, the composition ratio was 42.6% astaxanthin, 10.5% monoester, and less than 1% diester.

Example 6

Twenty-four mg of a mixture of astaxanthin esters extracted from the nature (manufactured by Itano, product name: Astax9000H) and 276 mg of tricaprilin as a triglyceride form fatty acid were placed in a brown glass bottle. Two hundred seventy mg of lipase derived from Candida (manufactured by Meito Sangyo Co., Ltd., product name: Lipase OF) was added thereto, and 22.5 μL of water was further added thereto and stirred fully afterwards. Thereafter, while stirring, reaction was carried out at 45°C. Three days later, the reaction solution was taken out, and the composition ratio of astaxanthin was analyzed by HPLC. As a result, the composition ratio was 55.9% astaxanthin, 25.0% monoester, and less than 1% diester.

Example 7

Two mg of a free astaxanthin (manufactured by Sigma) and 300 mg of octanoic acid as a free fatty acid were placed in a brown glass bottle. Three hundred mg of immobilized lipase prepared by the method described in the example of lipase immobilization was added thereto, and 22.5 μL of water was further added thereto and stirred fully afterwards. Thereafter, while shaking (170 rpm), reaction was carried out at 45°C. Three days later, the reaction product was taken out, and the composition ratio of astaxanthin was analyzed by HPLC. As a result, the composition ratio was 95.8% astaxanthin, 4.2% monoester, and less than 1% diester.

Example 8

Two mg of a free astaxanthin (manufactured by Sigma) and 300 mg of tricaprilin as a triglyceride form fatty acid were placed in a brown glass bottle. Thirty μL of water was added thereto and stirred fully afterwards. Thereafter, 300 mg of immobilized lipase prepared by the method described in the example of lipase immobilization was added thereto, and while shaking (170 rpm), reaction was carried out at 45°C. Three days later, the reaction product was taken out, and the composition ratio of astaxanthin was analyzed by HPLC. As a result, the composition ratio was 69.6% astaxanthin, 21.9% monoester, and 2.6% diester.

Example 9

Twenty-four mg of a mixture of astaxanthin esters extracted from the nature (manufactured by Itano, product name: Astax9000H) and 276 mg of octanoic acid as a free fatty acid were placed in a brown glass bottle. Three hundred mg of immobilized lipase prepared by the method described in the example of lipase immobilization was added thereto, and 30 μL of water was further added thereto and stirred fully afterwards. Thereafter, while stirring, reaction was carried out at 45°C. Four days later, the reaction product was taken out, and the composition ratio of astaxanthin was analyzed by HPLC. As a result, the composition ratio was 23.6% astaxanthin, 2.0% monoester, and less than 1% diester.

Example 10

Twenty-four mg of a mixture of astaxanthin esters extracted from the nature (manufactured by Itano, product name: Astax9000H) and 276 mg of tricaprilin as a triglyceride form fatty acid were placed in a brown glass bottle. Three hundred mg of immobilized lipase prepared by the method described in the example of lipase immobilization was added thereto, and 30 μL of water was further added thereto and stirred fully afterwards. Thereafter, while stirring, reaction was carried out at 45°C. Four days later, the reaction product was taken out, and the composition ratio of astaxanthin was analyzed by HPLC. As a result, the composition ratio was 64.0% astaxanthin, 12.2% monoester, and less than 1% diester.

Example 11

Twenty-four mg of a mixture of astaxanthin esters extracted from the nature (manufactured by Itano, product name: Astax9000H) and 276 mg of tricaprilin as a triglyceride form fatty acid were placed in a brown glass bottle. Eighty mg of lipase derived from Alcaligenes (manufactured by Meito Sangyo Co., Ltd., product name: Lipase PL) was added thereto, and 15 μL of water was further added thereto and stirred fully afterwards. Thereafter, while stirring, reaction was carried out at 45°C. Three days later, the reaction solution was taken out, and the composition ratio of astaxanthin was analyzed by HPLC. As a result, the composition ratio was 79.3% astaxanthin, 4.0% monoester, and less than 1%

diester.

Example 12

Twenty-four mg of a mixture of astaxanthin esters extracted from the nature (manufactured by Itano, product name: Astax9000H) and 276 mg of tricaprilin as a triglyceride form fatty acid were placed in a brown glass bottle. Fifteen μL of water was added thereto and stirred fully afterwards. Thereafter, 300 mg of the immobilized lipase PL prepared by the method described in the example of lipase immobilization was added thereto, and while stirring, reaction was carried out at 45°C. Three days later, the reaction product was taken out, and the composition ratio of astaxanthin was analyzed by HPLC. As a result, the composition ratio was 23.9% astaxanthin, 1% monoester, and less than 1% diester.

Example 13

Two mg of a free astaxanthin (manufactured by Sigma) and 300 mg of tricaprilin as a triglyceride form fatty acid were placed in a brown glass bottle. Three hundred mg of the immobilized lipase, Novozym435 (manufactured by Novozymes, Japan Ltd.), obtained by immobilizing lipase derived from Candida was added thereto, and 30 μL of water was further added thereto and stirred fully afterwards. Thereafter, while stirring, reaction was carried out at 45°C. Three days later, the reaction solution was taken out, and the composition ratio of astaxanthin was analyzed by HPLC. As a result, the composition ratio was 99.0% astaxanthin, 1.0% monoester, and less than 1% diester.

Example 14

Two mg of a free astaxanthin (manufactured by Sigma) and 300 mg of tricaprilin as a triglyceride form fatty acid were placed in a brown glass bottle. Three hundred mg of immobilized lipase prepared by the method described in the example of lipase immobilization was added thereto, and 30 μL of water and 12 ml of n-hexane were further added thereto and stirred fully afterwards. Thereafter, while stirring, reaction was carried out at 45°C. Three days later, the reaction product was taken out. The immobilized enzyme was eliminated by decantation, and the supernatant hexane was eliminated. The composition ratio of astaxanthin was then analyzed by HPLC. As a result, the composition ratio was 64.1% astaxanthin, 23.9% monoester, and 2.3% diester.

Example 15

Twenty-four mg of a mixture of astaxanthin esters extracted from the nature (manufactured by Itano, product name: Astax9000H) and 276 mg of tricaprilin as a triglyceride form fatty acid were placed in a brown glass bottle. Twelve ml of n-hexane was added thereto, and 300 mg of immobilized lipase prepared by the method described in the example of lipase immobilization and 30 μL of water were further added thereto and stirred fully afterwards. Thereafter, while stirring, reaction was carried out at 45°C. Three days later, the reaction product was taken out. The immobilized enzyme was eliminated by decantation, and the supernatant hexane was eliminated. The composition ratio of astaxanthin was then analyzed by HPLC. As a result, the composition ratio was 15.2% astaxanthin and 1% monoester.

Example 16

Two mg of a free astaxanthin (manufactured by Sigma) and 300 mg of lauric acid (C12:0) as a free fatty acid were placed in a brown glass bottle. In the above described examples, an astaxanthin and a fatty acid were in a solution state in this stage. However, since lauric acid is a solid at a lower than reaction temperature, these reaction materials were in a powdery state in this stage. Thus, twelve ml of n-hexane was added to the reaction materials, so that the materials became a solution. Thereafter, 300 mg of immobilized lipase prepared by the method described in the example of lipase immobilization and 30 μL of water were further added thereto, so that the materials finally became reactive. Reaction was carried out at 45°C, while stirring. Three days later, the reaction product was taken out. The immobilized enzyme was eliminated by decantation, and the supernatant hexane was eliminated. The composition ratio of astaxanthin was then analyzed by HPLC. As a result, the composition ratio was 93.6% astaxanthin, 6.4% monoester, and less than 1% diester.

Example 17

A mixture of two types of medium-chain fatty acid triglycerides (C8:0: tricaprilin and C10:0: tricaprin) is commercially available, and it can be used as a reaction material. Thus, this mixed medium-chain triglyceride was prepared at a ratio of 1 : 1 as a reagent. Then, two types of medium-chain fatty acid ester forms were synthesized from a free

astaxanthin. That is to say, 2 mg of a free astaxanthin (manufactured by Sigma), 300 mg of tricaprilin and 300 mg of tricaprin were placed in a brown glass bottle. Eighty mg of lipase derived from Candida (manufactured by Meito Sangyo Co., Ltd., product name: Lipase OF) was added thereto, and 60 μL of water was further added thereto and stirred fully afterwards. Thereafter, while stirring, reaction was carried out at 45°C. Three days later, the reaction product was taken out, and the composition ratio of astaxanthin was analyzed by HPLC. As a result, the composition ratio was 93.1% astaxanthin, 3.9% C8 monoester, 3.0% C10 monoester, and less than 1% diester.

Example 18

A mixture of two types of medium-chain fatty acid triglycerides (C8:0: tricaprilin and C10:0: tricaprin) is commercially available, and it can be used as a reaction material. Thus, this mixed medium-chain triglyceride was prepared at a ratio of 1 : 1 as a reagent. Then, two types of medium-chain fatty acid ester forms were synthesized from a free astaxanthin. That is to say, 2 mg of a free astaxanthin (manufactured by Sigma), 300 mg of tricaprilin and 300 mg of tricaprin were placed in a brown glass bottle. Three hundred mg of immobilized lipase prepared by the method described in the example of lipase immobilization was added thereto, and 30 μL of water was further added thereto and stirred fully afterwards. Thereafter, while shaking, reaction was carried out at 45°C. Three days later, the reaction product was taken out, and the composition ratio of astaxanthin was analyzed by HPLC. As a result, the composition ratio was 86.7% astaxanthin, 7.2% C8 monoester, 6.1% C10 monoester, and less than 1% diester.

Example 19

A mixture of two types of medium-chain fatty acid triglycerides (C8:0: tricaprilin and C10:0: tricaprin) is commercially available, and it can be used as a reaction material. Thus, this mixed medium-chain triglyceride was prepared at a ratio of 1 : 1 as a reagent. Then, two types of medium-chain fatty acid ester forms were synthesized in hexane from a free astaxanthin. That is to say, 2 mg of a free astaxanthin (manufactured by Sigma), 300 mg of tricaprilin and 300 mg of tricaprin were placed in a brown glass bottle. Twelve ml of n-hexane was added thereto, and three hundred mg of immobilized lipase prepared by the method described in the example of lipase immobilization and 30 μL of water were further added thereto and stirred fully afterwards. Thereafter, while stirring, reaction was carried out at 45°C. Three days later, the reaction product was taken out, and the composition ratio of astaxanthin was analyzed by HPLC. As a result, the composition ratio was 81.4% astaxanthin, 9.5% C8 monoester, 9.1% C10 monoester, and less than 1% diester.

Example 20

Forty mg of a mixture of astaxanthin esters extracted from the nature (manufactured by Itano, product name: Astax9000H) and 1.5 ml of tricaprilin as a triglyceride form fatty acid were placed in a brown glass bottle. Ninety μL of water was added thereto and the mixture was fully stirred. Thereafter, 300 mg of the immobilized lipase of Phenyl Toyopearl prepared by the method described in the example of lipase immobilization was further added thereto, and while stirring, reaction was carried out at 45°C. Four days later, the reaction product was taken out, and the composition ratio of astaxanthin was analyzed by HPLC. As a result, the composition ratio was 61.6% astaxanthin, 14.1% monoester, and 2.8% diester.

Example 21

Two mg of a free astaxanthin (manufactured by Sigma) and 300 mg of tricaprilin as a triglyceride form fatty acid were placed in a brown glass bottle. Thirty μL of water was added thereto and stirred fully afterwards. Thereafter, 300 mg of the immobilized lipase with HP 20 prepared by the method described in the example of lipase immobilization was added thereto, and while shaking (170 rpm), reaction was carried out at 45°C. Three days later, the reaction product was taken out, and the composition ratio of astaxanthin was analyzed by HPLC. As a result, the composition ratio was 96.1% astaxanthin and 3.9% monoester.

Example 22

Forty mg of a mixture of astaxanthin esters extracted from the nature (manufactured by Itano, product name: Astax9000H) and 1.5 ml of tricaprilin as a triglyceride form fatty acid were placed in a brown glass bottle. Ninety μL of water was added thereto and the mixture was fully stirred. Thereafter, 300 mg of the immobilized lipase with Hyflo supercel prepared by the method described in the example of lipase immobilization was further added thereto, and while stirring, reaction was carried out at 45°C. Four days later, the reaction product was taken out, and the composition ratio of astaxanthin was analyzed by HPLC. As a result, the composition ratio was 28.6% astaxanthin, 1.8% monoester, and

less than 1% diester.

Example 23

Twenty-four mg of a mixture of astaxanthin esters extracted from the nature (manufactured by Itano, product name: Astax9000H) and 1.3 ml of tricaprilin as a triglyceride form fatty acid were placed in a brown glass bottle. Ninety μL of water was added thereto and the mixture was fully stirred. Thereafter, 300 mg of the HPA25 resin-immobilized lipase, which was prepared by the method described in the example of lipase immobilization, was further added thereto. Thereafter, while stirring, reaction was carried out at 45°C. Three days later, the reaction product was taken out, and the composition ratio of astaxanthin was analyzed by HPLC. As a result, the composition ratio was 39.3% astaxanthin, 2.9% monoester, and less than 1% diester.

Example 24

Two mg of a free astaxanthin (manufactured by Sigma) and 1.3 g of tricaprilin as a triglyceride form fatty acid were placed in a brown glass bottle. Ninety μL of water was added thereto and stirred fully afterwards. Thereafter, 300 mg of the HPA25 resin-immobilized lipase, which was prepared by the method described in the example of lipase immobilization was added thereto, and while shaking (170 rpm), reaction was carried out at 45°C. Three days later, the reaction product was taken out, and the composition ratio of astaxanthin was analyzed by HPLC. As a result, the composition ratio was 79.8% astaxanthin, 19.2% monoester, and 1.0% diester.

Example 25

Twenty-four mg of a mixture of astaxanthin esters extracted from the nature (manufactured by Itano, product name: Astax9000H) and 1.3 ml of tricaprilin as a triglyceride form fatty acid were placed in a brown glass bottle. Ninety μL of water was added thereto and the mixture was fully stirred. Thereafter, 300 mg of the HP1MG resin-immobilized lipase, which was prepared by the method described in the example of lipase immobilization, was further added thereto. Thereafter, while stirring, reaction was carried out at 45°C. Three days later, the reaction product was taken out, and the composition ratio of astaxanthin was analyzed by HPLC. As a result, the composition ratio was 54.9% astaxanthin, 5.4% monoester, and less than 1% diester.

Example 26

Two mg of a free astaxanthin (manufactured by Sigma) and 1.3 g of tricaprilin as a triglyceride form fatty acid were placed in a brown glass bottle. Ninety μL of water was added thereto and stirred fully afterwards. Thereafter, 300 mg of the HP1MG resin-immobilized lipase, which was prepared by the method described in the example of lipase immobilization was added thereto, and while shaking (170 rpm), reaction was carried out at 45°C. Three days later, the reaction product was taken out, and the composition ratio of astaxanthin was analyzed by HPLC. As a result, the composition ratio was 79.5% astaxanthin, 18.7% monoester, and 1.8% diester.

The results of the above examples are summarized in Tables 2 to 6. These results show that the transesterification method using lipase of the present invention can effectively synthesize astaxanthin medium-chain fatty acid esters.

(Table 2)

| | | Examples | | | | | |
|---|---|---|---|---|---|---|---|
| | | 1 | 2 | 3 | 4 | 5 | 6 |
| Reaction conditions | Astaxanthin<br>Type and amount (mg) | Free<br>2 | Free<br>2 | OA ester<br>2 | OA ester<br>2 | Astax<br>24 | Astax<br>24 |
| | Fatty acid<br>Type and amount (mg) | C8 acid<br>300 | C8TG<br>300 | C8 acid<br>300 | C8TG<br>300 | C8 acid<br>276 | C8TG<br>276 |
| | Lipase<br>Type and amount (mg) | OF<br>80 | OF<br>120 | OF<br>80 | OF<br>80 | OF<br>270 | OF<br>270 |
| | Carrier of lipase | None | None | None | None | None | None |
| | Additive amount of water (%) | 10 | 10 | 10 | 10 | 7.5 | 7.5 |
| | Solvent<br>Type and amount | None | None | None | None | None | None |
| Reaction products | Astaxanthin (monoester) | 6.0 | 13.6 | 8.2 | 15.3 | 10.5 | 25.0 |
| | Astaxanthin (diester) | <1.0 | 1.5 | <1.0 | <1.0 | <1.0 | <1.0 |
| | Astaxanthin (free type) | 94.0 | 84.9 | 61.1 | 73.4 | 42.6 | 55.9 |

(Table 3)

| | | Examples | | | | | |
|---|---|---|---|---|---|---|---|
| | | 7 | 8 | 9 | 10 | 11 | 12 |
| Reaction conditions | Astaxanthin Type and amount (mg) | Free 2 | Free 2 | Astax 24 | Astax 24 | Astax 24 | Astax 24 |
| | Fatty acid Type and amount (mg) | C8 acid 300 | C8TG 300 | C8 acid 276 | C8TG 276 | C8TG 300 | C8TG 276 |
| | Lipase Type and amount (mg) | OF 300 | OF 300 | OF 300 | OF 300 | PL 80 | PL 300 |
| | Carrier of lipase | Ion exchange resin | Ion exchange resin | Ion exchange resin | Ion exchange resin | None | Ion exchange resin |
| | Additive amount of water (%) | 7.5 | 3.3 | 10 | 10 | 5 | 5 |
| | Solvent Type and amount | None | None | None | None | None | None |
| Reaction products | Astaxanthin (monoester) | 4.2 | 21.9 | 2.0 | 12.2 | 4.0 | 1.0 |
| | Astaxanthin (diester) | <1.0 | 2.6 | <1.0 | <1.0 | <1.0 | <1.0 |
| | Astaxanthin (free type) | 95.8 | 69.6 | 23.6 | 64.0 | 79.3 | 23.9 |

(Table 4)

| | | Examples | | | | | |
|---|---|---|---|---|---|---|---|
| | | 13 | 14 | 15 | 16 | 17 | 18 |
| Reaction conditions | Astaxanthin Type and amount (mg) | Free 2 | Free 2 | Astax 24 | Free 2 | Free 2 | Free 2 |
| | Fatty acid Type and amount (mg) | C8TG 300 | C8TG 300 | C8TG 276 | C12 acid 300 | TG (C8+C10) 600 | TG (C8+C10) 300 |
| | Lipase Type and amount (mg) | Novo 435 300 | OF 300 | OF 300 | OF 300 | OF 80 | OF 300 |
| | Carrier of lipase | Celite | Ion exchange resin | Ion exchange resin | Ion exchange resin | None | Ion exchange resin |
| | Additive amount of water (%) | 10 | 10 | 10 | 10 | 60 | 10 |
| | Solvent Type and amount | None | n-Hexane 12 | n-Hexane 12 | n-Hexane 12 | n-Hexane 12 | None |
| Reaction products | Astaxanthin (monoester) | 1.0 | 23.9 | 1.0 | 6.4 | C8:3.9 C10:3.0 | C8:7.2 C10:6.1 |
| | Astaxanthin (diester) | <1.0 | 2.3 | <1.0 | <1.0 | <1.0 | <1.0 |
| | Astaxanthin (free type) | 99.0 | 64.1 | 15.2 | 93.6 | 93.1 | 86.7 |

(Table 5)

| | | Examples | | | | | |
|---|---|---|---|---|---|---|---|
| | | 19 | 20 | 21 | 22 | 23 | 24 |
| Reaction conditions | Astaxanthin<br>Type and amount (mg) | Free<br>2 | Astax<br>40 | Free<br>2 | Astax<br>40 | Astax<br>40 | Free<br>2 |
| | Fatty acid<br>Type and amount (mg) | TG<br>(C8+C10)<br>300 | C8TG<br>1500 | C8TG<br>300 | C8TG<br>1500 | C8TG<br>1300 | C8TG<br>1300 |
| | Lipase<br>Type and amount (mg) | OF<br>300 | OF<br>300 | OF<br>300 | OF<br>300 | OF<br>300 | OF<br>300 |
| | Carrier of lipase | Ion exchange resin | Phenyl Toyoyearl resin | HP20 resin | Celite | HPA25 resin | HPA25 resin |
| | Additive amount of water (%) | 10 | 6 | 10 | 6 | 7 | 7 |
| | Solvent<br>Type and amount | n-Hexane<br>12 | None | None | None | None | None |
| Reaction products | Astaxanthin (monoester) | C8:9.5<br>C10:9.1 | 14.1 | 3.9 | 1.6 | 2.9 | 19.2 |
| | Astaxanthin (diester) | <1.0 | 2.8 | <1.0 | <1.0 | <1.0 | 1.0 |
| | Astaxanthin (free type) | 81.4 | 61.6 | 96.1 | 28.6 | 39.3 | 79.8 |

(Table 6)

| | | Examples | | | | | |
|---|---|---|---|---|---|---|---|
| | | 25 | 26 | | | | |
| Reaction conditions | Astaxanthin Type and amount (mg) | Astax 40 | Astax 40 | | | | |
| | Fatty acid Type and amount (mg) | C8TG 1300 | C8TG 1300 | | | | |
| | Lipase Type and amount (mg) | OF 300 | OF 300 | | | | |
| | Carrier of lipase | HP1MG resin | HP1MG resin | | | | |
| | Additive amount of water (%) | 7 | 7 | | | | |
| | Solvent Type and amount | None | None | | | | |
| Reaction products | Astaxanthin (monoester) | 5.4 | 18.7 | | | | |
| | Astaxanthin (diester) | <1.0 | 1.8 | | | | |
| | Astaxanthin (free type) | 54.9 | 79.5 | | | | |

Example 27 Extraction, fractionation and purification of astaxanthin esters from natural products

A commercially available Euphausiacea (356.3 g) was disrupted in a mortar, using a pestle. Then, the Euphausiacea was extracted three times with 5 times amount of acetone followed by vacuum concentration. Thereafter, extraction was carried out three times using a saturated saline solution and ethyl acetate, so that a crude extract (52.13 g) was obtained from the ethyl acetate layer. The obtained Euphausiacea extract was subjected to column chromatography in the following order:

1) 1st silica column chromatography

Using silica gel (Silica gel 60 manufactured by Merck, 500 g), column chromatography was carried out. Stepwise elution was carried out with an elution solvent of hexane/acetone in the order of the concentrations (90 : 10, 85 : 15, 80 : 20, 75 : 25, 70 : 30, 60 : 40, 50 : 50, 40 : 60, 30 : 70, 20 : 80, 10 : 90, 0 : 100). Elution with the same solvent system was carried out by successively adding an elution solvent in an amount three times volume of the column. After each eluate was concentrated under a reduced pressure, an astaxanthin monoester fraction (116.32 mg) was obtained.

2) ODS column chromatography

Using ODS gel (ODS-SS-1020T manufactured by Senshu Scientific Co., Ltd., 50 g), column chromatography was carried out. Using 100% methanol as an elution solvent, a dark red fraction eluted from the column was dispensed and concentrated under a reduced pressure, and an astaxanthin monoester fraction (8.84 mg) was obtained.

3) 2nd silica column chromatography

Using silica gel (Silica gel 60 manufactured by Merck, 30 g), column chromatography was carried out. Using dichloromethane/ethyl acetate (8 : 2) as an elution solvent, a dark red fraction eluted from the column was dispensed and concentrated under a reduced pressure, and an astaxanthin monoester fraction (2.87 mg) was obtained.

4) 3rd silica column chromatography

Using silica gel (Silica gel 60 manufactured by Merck, 30 g), column chromatography was carried out. Using hexane/acetone (7 : 3) as an elution solvent, a dark red fraction eluted from the column was dispensed and concentrated under a reduced pressure, and an astaxanthin monoester fraction (0.48 mg) with high purity was obtained.

Example 28 Analysis of astaxanthin ester fraction

An astaxanthin ester fraction from the Euphausiacea extract prepared in Example 27 was dissolved in 1 ml of methanol, and while stirring at 80°C, 0.5 ml of 1 mol/l sodium methoxide was added thereto by dropwisely. Extraction was carried out using ethyl acetate and water to eliminate alkali contained in the reaction product. The ethyl acetate layer was subjected to vacuum concentration, and fatty acid analysis was carried out using GC-MS (G1800A manufactured by Hewlett-Packard). Moreover, in order to confirm whether or not a free fatty acid was mixed in the astaxanthin monoester fraction, the GC-MS analysis was carried out without carrying out methyl esterification.

"Measurement method data"

Injection temperature: 200°C, Detection temperature: 300°C, Initial column temperature: 40°C, Increased temperature: 2.5°C/min., Column size: 30.0 m x 0.25 mm, Gas: helium gas, Flow rate of gas: 1.0 ml/min., Mass range: 45 : 200 m/z, waiting time for solvent: 5 minutes

The GC-MS data of authentic octanoic acid methyl ester are shown in Figure 3, and the GC-MS data of Euphausiacea-derived astaxanthin monoester samples are shown in Figure 4. As shown in these figures, both data completely match with each other. No parent ion peak (m/z 158) was observed in either the specimens or the samples, but a specific fragmentation pattern (m/z 127) was observed, and at the same time m/z 55, m/z 59 and m/z 87 derived from a fatty acid methyl esterified product were observed. Moreover, when samples that were not methyl esterified were also analyzed, no corresponding peaks were observed, thereby confirming that no free octanoic acid and no octanoic acid methyl ester were present in the sample.

From the above results, it was confirmed that an astaxanthin octanoic acid ester is present in Euphausiacea.

Examples of application to cosmetics

A lipstick was produced by the following method, using a composition containing a 1% astaxanthin octanoic acid monoester, which was obtained by carrying out enzyme reaction and then carrying out purification operation.

The below-indicated oily substrates were mixed, and the mixture was dissolved by heating. A pigment (50 mg) and a composition (150 mg) containing a 1% astaxanthin octanoic acid monoester were fully mixed with castor oil (2.1 g) in advance. The above dissolved and dispersed product was added to the mixture followed by stirring. A perfume (150 mg) and an antioxidant (50 mg) were added thereto, and the mixture was further stirred and mixed, so as to make the mixture homogenous. The obtained liquid was poured into a mold followed by quenching. The lipstick obtained by cooling was placed in a container, and the surface was heated with a small burner in a short time to put a shine thereon, so as to obtain a lipstick.

| Mixing ratio of oily substrates | |
|---|---|
| Beeswax | 1.0 g |
| Ceresin | 2.4 g |
| Carnauba wax | 0.8 g |
| Lanolin | 1.0 g |
| Liquid paraffin | 2.05 g |
| Eosin acid | 0.25 g |

Examples of application to food

Soft capsule used for healthy food and/or supplements

A soft capsule was produced by the following method, using a composition containing a 10% astaxanthin octanoic acid monoester, which was obtained by carrying out enzyme reaction and then carrying out purification operation.

A substrate was prepared at the following ratio, and a composition (30 mg) containing a 10% astaxanthin octanoic acid monoester was added to the substrate, so that a soft capsule used for a healthy food and/or supplements was prepared. Preparation ratio (amount per capsule)
Substrate (plant oil): 130 mg
Emulsifier (beeswax): 30 mg
Coating material (zelatin/glycerine = 100/35): 150 mg

Examples of the use as food material

An example of preparing an astaxanthin medium-chain fatty acid ester-containing oils and fats as food materials in order to use them for food, cosmetics, feed and others is described below.

The method described in Example 14 regarding enzyme reaction was further scaled up. Two gram of a material free astaxanthin was reacted with 300 g of a medium-chain fatty acid triglyceride, and after the elimination of hexane, approximately 300 g of oils and fats were obtained as a result of the reaction. Caprylic acid released during the reaction was distilled by molecular distillation at 130°C, 0.2mmHg, so as to obtain 10 g of an astaxanthin medium-chain fatty acid ester-containing oils and fats. The obtained oils and fats were subjected to a steam distillation, and thereafter, 10 g of a medium-chain fatty acid triglyceride was added thereto, so as to obtain 3% astaxanthin medium-chain fatty acid ester-containing oils and fats. The total astaxanthins had a concentration of 10%. The oils and fats were defined as food materials (astaxanthin medium-chain fatty acid ester-containing oils and fats), which are used for various purposes.

Improvement of digestibility of astaxanthin

Using a commercially available astaxanthin extracted from Chlorophyceae of Haematococcus (Itano, product name: Astax9000H) and astaxanthins mono- and di-esterified with a medium-chain fatty acid (Asta-C8-monoester and Asta-C8-diester), the digestibility of these astaxanthins was studied using rats. For the experiment, these astaxanthins were diluted with olive oil so as to obtain a ratio of 100 mg/kg in the conversion of a free astaxanthin, and the diluted astaxanthins were administered to rats (Wister rat). The content of astaxanthin contained in the blood (blood plasma) and the liver of each rat was measured using HPLC. The blood and liver were sampled at 3, 5, 7 and 10 hours after the administration. Figure 1 shows the amount of the astaxanthin taken in the blood plasma, and Figure 2 shows that taken in the liver. As shown in Figures 1 and 2, when compared with the astaxanthin extracted from Chlorophyceae of Haematococcus, the astaxanthin medium-chain fatty acid esters showed much better digestibility. In particular, the monoester form of the astaxanthin medium-chain fatty acid esters was most digested. Further, the administered ester form astaxanthin was detected as a free astaxanthin.

Therefore, it was found that the astaxanthin medium-chain fatty acid monoester is an astaxanthin having excellent digestibility.

INDUSTRIAL APPLICABILITY

According to the method of producing an astaxanthin medium-chain fatty acid ester of the present invention, reaction can be carried out under mild condition, using lipase, and the astaxanthin medium-chain fatty acid ester can be produced at a high yield without inducing the decomposition or isomerization of a material astaxanthin. Moreover, the method of the present invention can extract and produce the astaxanthin medium-chain fatty acid ester from natural products.

Furthermore, if a composition comprising the astaxanthin medium-chain fatty acid ester of the present invention is added to a food, a healthy food and/or supplements or cosmetics, it can provide those containing an astaxanthin having excellent digestibility and tissue penetration.

## Claims

1. An astaxanthin medium-chain fatty acid ester selected from astaxanthin octanoic acid monoester, astaxanthin octanoic acid diester and astaxanthin decanoic acid monoester.

2. The astaxanthin medium-chain fatty acid ester according to claim 1, wherein the astaxanthin medium-chain fatty acid ester is astaxanthin octanoic acid monoester or astaxanthin octanoic acid diester.

3. The astaxanthin medium-chain fatty acid ester according to claim 1, wherein the astaxanthin medium-chain fatty acid ester is astaxanthin octanoic acid monoester or astaxanthin decanoic acid monoester.

4. The astaxanthin medium-chain fatty acid ester according to claim 1 or 2, wherein the astaxanthin medium-chain fatty acid ester is astaxanthin octanoic acid diester.

5. A composition comprising the astaxanthin medium-chain fatty acid ester according to any of claims 1 to 4.

6. The composition according to claim 5, which comprises the astaxanthin medium-chain fatty acid ester in an amount of at least 0.1%.

7. A food composition obtainable by mixing the food and the composition according to claim 5 or 6.

8. A food additive, a cosmetic, or an animal feed, which comprises the composition according to claim 5 or 6.

9. A method of producing the astaxanthin medium-chain fatty acid ester according to any one of claims 1 to 4, wherein said method comprises the step of lipase reaction.

10. The method according to claim 9, wherein an esterification and/or transesterification is carried out using one or more astaxanthin materials selected from the group consisting of a free astaxanthin, an ester form astaxanthin different from a medium-chain fatty acid ester, and a mixture of ester form astaxanthins different from a medium-chain fatty acid ester; and one or more medium-chain fatty acid materials selected from the group consisting of a free medium-chain fatty acid, a medium-chain fatty acid monoglyceride, a medium-chain fatty acid diglyceride, a medium-chain fatty acid triglyceride, and a medium-chain fatty acid lower alcohol ester.

11. The method according to claim 9 or 10, wherein the lipase is one or more of lipases selected from the group consisting of a lipase derived from yeast belonging to Candida, lipase derived from a microorganism belonging to Chromobacterium, a lipase derived from a microorganism belonging to Alcaligenes, and a lipase derived from animal pancreas.

12. The method according to claim 11, wherein the lipase is derived from yeast belonging to Candida.

13. The method according to any one of claims 10 to 12, wherein the astaxanthin material is free astaxanthin and/or a mixture of different types of ester form astaxanthins, and the medium-chain fatty acid material is a medium-chain fatty acid triglyceride.

14. The method according to any one of claims 9 to 13, wherein water is added.

15. The method according to claim 14, wherein water is added at the amount of 0.5 w/w to 20 w/w % with respect to the amount of the oil material.

16. A method of producing astaxanthin octanoic acid monoester or astaxanthin octanoic acid diester, which comprises the following steps (a) and (b):

(a) a step of extracting said compound from Crustacea using a solvent or supercritical $CO_2$, and
(b) a step of purifying said compound from the extract obtained by the step (a).

17. The method according to claim 16, wherein Crustacea is Euphausiacea.

**Patentansprüche**

1. Astaxanthin-Fettsäureester mit mittlerer Kettenlänge, der aus Astaxanthin-Octansäuremonoester, Astaxanthin-Octansäurediester und Astaxanthin-Decansäuremonoester ausgewählt ist.

2. Astaxanthin-Fettsäureester mit mittlerer Kettenlänge gemäß Anspruch 1, wobei der Astaxanthin-Fettsäureester mit mittlerer Kettenlänge Astaxanthin-Octansäuremonoester oder Astaxanthin-Octansäurediester ist.

3. Astaxanthin-Fettsäureester mit mittlerer Kettenlänge gemäß Anspruch 1, wobei der Astaxanthin-Fettsäureester mit mittlerer Kettenlänge Astaxanthin-Octansäuremonoester oder Astaxanthin-Decansäuremonoester ist.

4. Astaxanthin-Fettsäureester mit mittlerer Kettenlänge gemäß Anspruch 1 oder 2, wobei der Astaxanthin-Fettsäureester mit mittlerer Kettenlänge Astaxanthin-Octansäurediester ist.

5. Zusammensetzung umfassend den Astaxanthin-Fettsäureester mit mittlerer Kettenlänge gemäß einem der Ansprüche 1 bis 4.

6. Zusammensetzung gemäß Anspruch 5, welche den Astaxanthin-Fettsäureester mit mittlerer Kettenlänge in einer Menge von mindestens 0,1% umfasst.

7. Nahrungsmittelzusammensetzung, erhältlich durch Mischen des Nahrungsmittels und der Zusammensetzung gemäß Anspruch 5 oder 6.

8. Nahrungsmittelzusatz, Kosmetikum oder Tierfutter, welcher/welches die Zusammensetzung gemäß Anspruch 5 oder 6 umfasst.

9. Verfahren zur Herstellung des Astaxanthin-Fettsäureesters mit mittlerer Kettenlänge gemäß einem der Ansprüche 1 bis 4, wobei das Verfahren den Schritt der Lipasereaktion umfasst.

10. Verfahren gemäß Anspruch 9, wobei eine Veresterung und/oder Umesterung ausgeführt wird unter Verwendung eines oder mehrerer Astaxanthin-Materialien, die aus freiem Astaxanthin, Astaxanthin in Form eines Esters, der sich von einem Fettsäureester mit mittlerer Kettenlänge unterscheidet, und einem Gemisch von Astaxanthinen in Form von Estern, die sich von einem Fettsäureester mit mittlerer Kettenlänge unterscheiden, ausgewählt sind; und eines oder mehrerer Fettsäurematerialien mit mittlerer Kettenlänge, die aus einer freien Fettsäure mit mittlerer Kettenlänge, einem Fettsäuremonoglycerid mit mittlerer Kettenlänge, einem Fettsäurediglycerid mit mittlerer Kettenlänge, einem Fettsäuretriglycerid mit mittlerer Kettenlänge und einem Ester einer Fettsäure mit mittlerer Kettenlänge und einem niederen Alkohol ausgewählt sind.

11. Verfahren gemäß Anspruch 9 oder 10, wobei die Lipase eine oder mehrere der Lipasen ist, die aus einer Lipase, die sich von einer zu Candida gehörenden Hefe ableitet, einer Lipase, die sich von einem zu Chromobacterium gehörenden Mikroorganismus ableitet, einer Lipase, die sich von einem zu Alcaligenes gehörenden Mikroorganismus ableitet, und einer Lipase, die sich von einer tierischen Bauchspeicheldrüse ableitet, ausgewählt sind.

12. Verfahren gemäß Anspruch 11, wobei sich die Lipase von einer zu Candida gehörenden Hefe ableitet.

13. Verfahren gemäß einem der Ansprüche 10 bis 12, wobei das Astaxanthinmaterial freies Astaxanthin und/oder ein Gemisch von verschiedenen Arten von Astaxanthinen in Form eines Esters ist, und das Fettsäurematerial mit mittlerer Kettenlänge ein Fettsäuretrigylcerid mit mittlerer Kettenlänge ist.

14. Verfahren gemäß einem der Ansprüche 9 bis 13, wobei Wasser zugegeben wird.

15. Verfahren gemäß Anspruch 14, wobei Wasser in einer Menge von 0,5 bis 20 % Gew./Gew. bezogen auf die Menge des Ölmaterials zugegeben wird.

16. Verfahren zur Herstellung von Astaxanthin-Octansäuremonoester oder Astaxanthin-Octansäurediester, welches die folgenden Schritte (a) und (b) umfasst:

(a) einen Schritt zur Extraktion der Verbindung aus Crustacea unter Verwendung eines Lösungsmittels oder von überkritischen $CO_2$, und
(b) einen Schritt zur Reinigung der Verbindung aus dem Extrakt, das in Schritt (a) erhalten wird.

17. Verfahren gemäß Anspruch 16, wobei Crustacea Euphausiacea ist.

## Revendications

1. Ester d'acide gras à chaîne moyenne d'astaxanthine choisi parmi le monoester d'acide octanoïque d'astaxanthine, le diester d'acide octanoïque d'astaxanthine et le monoester d'acide décanoïque d'astaxanthine.

2. Ester d'acide gras à chaîne moyenne d'astaxanthine selon la revendication 1, dans lequel l'ester d'acide gras à chaîne moyenne d'astaxanthine est le monoester d'acide octanoïque d'astaxanthine ou le diester d'acide octanoïque d'astaxanthine.

3. Ester d'acide gras à chaîne moyenne d'astaxanthine selon la revendication 1,
dans lequel l'ester d'acide gras à chaîne moyenne d'astaxanthine est le monoester d'acide octanoïque d'astaxanthine ou le monoester d'acide décanoïque d'astaxanthine.

4. Ester d'acide gras à chaîne moyenne d'astaxanthine selon la revendication 1 ou 2, dans lequel l'ester d'acide gras à chaîne moyenne d'astaxanthine est le diester d'acide octanoïque d'astaxanthine.

5. Composition comprenant l'ester d'acide gras à chaîne moyenne d'astaxanthine selon l'une quelconque des revendications 1 à 4.

6. Composition selon la revendication 5, qui comprend l'ester d'acide gras à chaîne moyenne d'astaxanthine en une quantité d'au moins 0, 1 %.

7. Composition alimentaire susceptible d'être obtenue en mélangeant l'aliment et la composition selon la revendication 5 ou 6.

8. Additif alimentaire, cosmétique, ou aliment pour animaux, qui comprend la composition selon la revendication 5 ou 6.

9. Procédé de préparation de l'ester d'acide gras à chaîne moyenne d'astaxanthine selon l'une quelconque des revendications 1 à 4, dans lequel ledit procédé comprend une étape de réaction avec une lipase.

10. Procédé selon la revendication 9, dans lequel une estérification et/ou une transestérification est effectuée en utilisant un ou plusieurs matériaux dérivés de l'astaxanthine choisis dans le groupe consistant en l'astaxanthine libre, une d'astaxanthine sous forme d'un ester différent d'un ester d'acide gras à chaîne moyenne, et un mélange d'astaxanthines sous forme d'esters différents d'un ester d'acide gras à chaîne moyenne ; et un ou plusieurs matériaux dérivés d'un acide gras à chaîne moyenne choisis dans le groupe consistant en un acide gras à chaîne moyenne libre, un monoglycéride d'acide gras à chaîne moyenne, un diglycéride d'acide gras à chaîne moyenne, un triglycéride d'acide gras à chaîne moyenne, et un ester d'alcool inférieur d'acide gras à chaîne moyenne.

11. Procédé selon la revendication 9 ou 10, dans lequel la lipase est une ou plusieurs des lipases choisies dans le groupe consistant en une lipase dérivée d'une levure appartenant au genre Candida, une lipase dérivée d'un micro-organisme appartenant au genre Chromobacterium, une lipase dérivée d'un micro-organisme appartenant au genre Alcaligenes, et une lipase dérivée d'un pancréas animal.

12. Procédé selon la revendication 11, dans lequel la lipase est dérivée d'une levure appartenant au genre Candida.

13. Procédé selon l'une quelconque des revendications 10 à 12, dans lequel le matériau dérivé de l'astaxanthine est l'astaxanthine libre et/ou un mélange de différents types d'astaxanthines sous forme d'esters, et le matériau dérivé d'un acide gras à chaîne moyenne est un triglycéride d'acide gras à chaîne moyenne.

14. Procédé selon l'une quelconque des revendications 9 à 13, dans lequel de l'eau est ajoutée.

15. Procédé selon la revendication 14, dans lequel de l'eau est ajoutée en une quantité de 0,5% poids/poids à 20% poids/poids par rapport à la quantité de matériau de type huile.

16. Procédé de préparation du monoester d'acide octanoïque d'astaxanthine ou du diester d'acide octanoïque d'astaxanthine,
qui comprend les étapes suivantes (a) et (b):

(a) une étape d'extraction dudit composé provenant de Crustacea en utilisant un solvant ou du $CO_2$ supercritique, et
(b) une étape de purification dudit composé provenant de l'extrait obtenu selon l'étape (a).

**17.** Procédé selon la revendication 16, dans lequel Crustacea est Euphausiacea.

Figure 1 ASTAXANTHIN CONCENTRATION IN BLOOD PLASMA

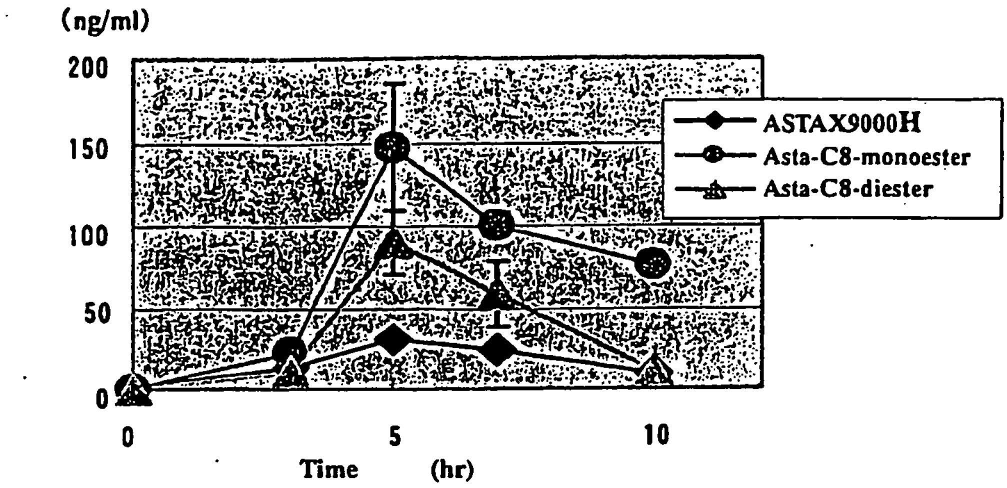


Figure 2 ASTAXANTHIN TRANSITED TO LIVER

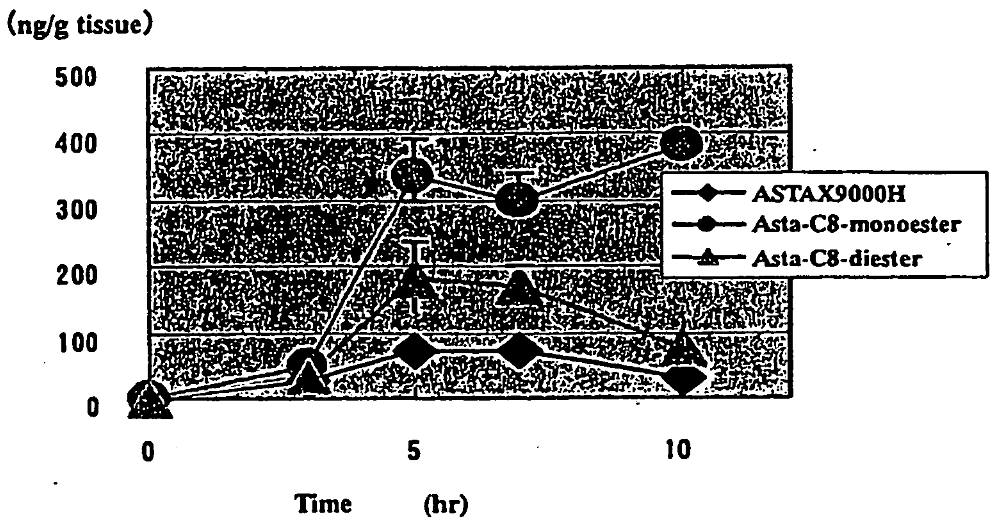

Figure 3 GC-MS DATA OF AUTHENTIC OCTANOIC ACID METHYL ESTER

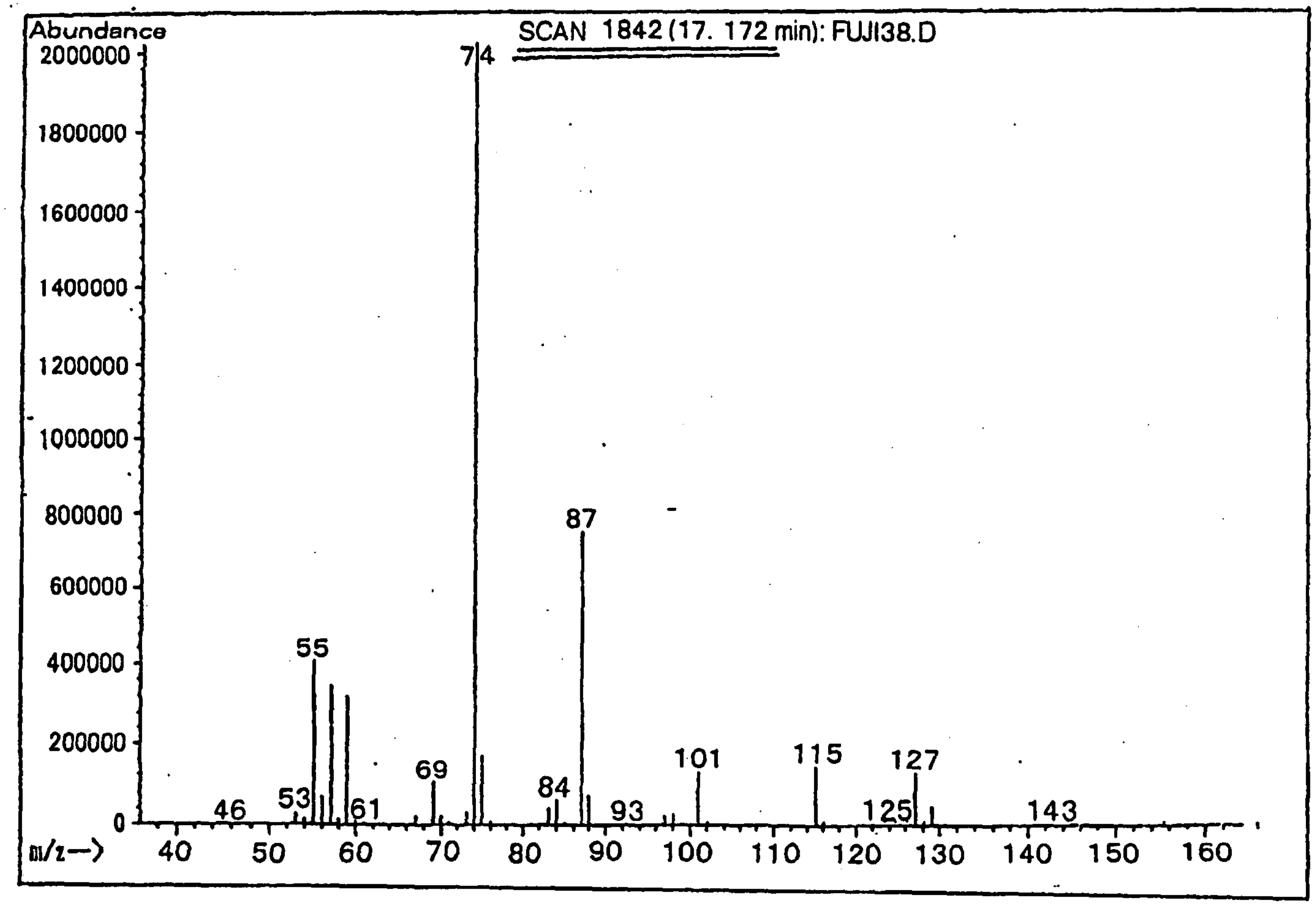

Figure 4 GC-MS DATA OF PURIFIED EUPHAUSIACEA SAMPLES

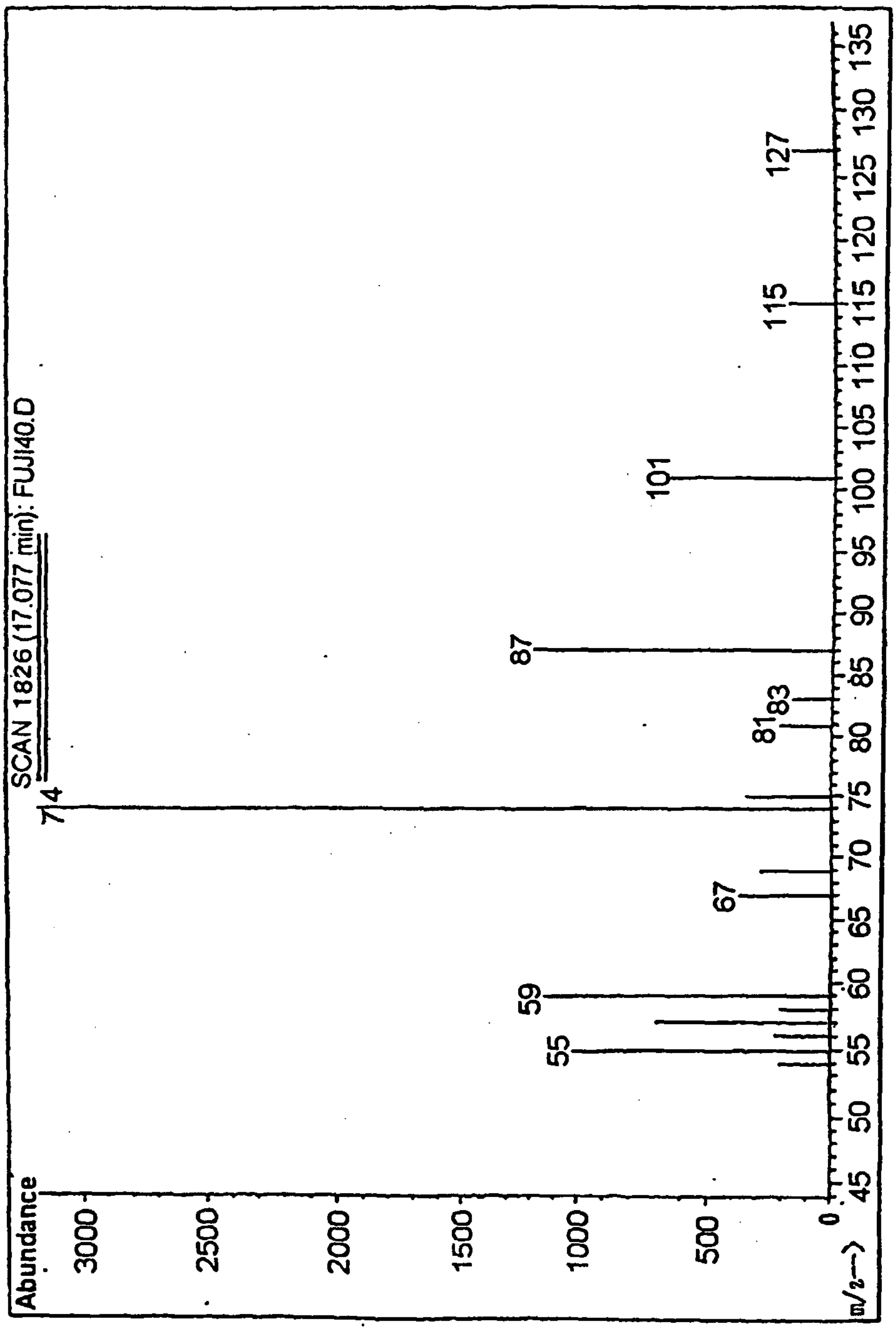

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 11290094 A
- JP 1202261 A


### Non-patent literature cited in the description


- *Shokuhin to Kaihatsu,* 1992, vol. 27 (3), 38-40
- *Kagaku to Seibutsu,* 1990, vol. 28 (4), 219-227
- *Comp. Biochem. Physiol., B: Comp. Biochem.,* 1987, vol. 86B (3), 587-591
- Bioreactor. Kodansha Scientific, 1985
- Jissen Bioreactor. 1990